# EUROPEAN PATENT APPLICATION

(11) **EP 1 612 267 A1**
(43) Date of publication of application: **04.01.2006**
(21) Application number: 04015680.4
(22) Date of filing: 02.07.2004
(51) Int. Cl.: C12N 9/42

(54) **Cellulases from rumen**

(71) Applicant: GBF Gesellschaft für Biotechnologische Forschung mbH, 38124 Braunschweig (DE); Vialactia Bioscience (NZ) Limited, Newmarket, Auckland (NZ)
(72) Inventor: Ferrer, Manuel, Dr., 28049 Madrid (ES); Golyshin, Peter, Dr., 38302 Wolfenbüttel (DE); Golyshina, Olga, Dr., 38302 Wolfenbüttel (DE); Chernikova, Tatyana, Dr., 38102 Braunschweig (DE); Strömpl, Carsten, 38173 Evessen (DE); Timmis, Kenneth, Prof. Dr., 38300 Wolfenbüttel (DE)
(74) Representative: Graf von Stosch, Andreas

(57) **Abstract**

The invention provides polypeptides coding for new cellulases from rumen, particularly from rumen ecosystem. The invention also relates to functional fragments or functional derivatives thereof as well as to nucleic acids encoding the polypeptides of the invention, vectors and host cells containing said nucleic acids, a method for producing the polypeptides and the use of the polypeptides according to the present invention for various industrial purposes and medical treatments.

SEQUENCE LISTING

<110> GBF Gesellschaft fuer Biotechnologische Forschung mbH, et
al.

<120> New cellulases from rumen

<130> GB01P002EP

<160> 21

<170> PatentIn version 3.3

<210> 1
<211> 1587
<212> DNA
<213> Unknown

<220>
<223> Description of sequence: sequence from genomic DNA library from
rumen ecosystem, particularly nucleic acid sequence of clones
pBKRR 1 and 21
; RR01-1 First enzyme (= RR21)
; CDS; (see Figure 9)

<400> 1
atgaaactgt attggaaata tctttcattg actgctgtgc tggctgtcct ggcttcctgc 60

ggcggcaatg ctgatcccga tccggagccg acgccctcgg cgccatcatc cattaccttg 120

agcgtcaatt cctttacagt tgcgcaggcc ggtgagaccc tgtccctgga cattacggct 180

cccactcgtc cgaaactcgc actcccgaac tggattactc tcaaggacgg tacctatcag 240

aattataaga ttaccgtggg actgacggtt tcggccaatg atacgtacca ggcacgtgag 300

gcgactgtga cggtttctgc cacggggact ttcgacgtta ccttcaaggt ttcccaggcc 360

gggaaagaac ctgaacccac gccgccggat ccgagcggcg gggacaacga tgcctggcag 420

atggcgaaga agttggggtt gggctggaac atgggcaatc atttcgatgc tttctacaat 480

ggttcctggg ccggtgagaa ggaaggctat ccggacgagg aggtctgggg tgcgtcgaag 540

gctacgcaga cgacattcaa tggcgtgaag aatgccggct ttacgagtgt ccgcatccct 600

gtctcctggc tgaagatgat cggtccggct ccggaataca agattgacga gacttggctg 660

aaccgcgtgt atgaggttgc gcagtatgcc cataatgccg gactgactgc gattgtcaat 720

acgcaccatg atgagaacca cggcgtggac aatacctatc agtggctgga catcaagaat 780

gcttcgaccg acgcttccgt gaatacgcgg atcaaggagg aaatcaaggc cgtctggacc 840

cagatcgcga acaagttcaa ggattgtgga gactggctca tcctggagag tttcaacgaa 900

ttgaacgacg gtggctgggg ctggagcgat gctttccggg ccaatccgac caaacagtgc 960

aatatcctga acgagtggaa ccaggtattc gtggatgcgg tccgtgccac cggcgggaac 1020

aatgccaccc gctggctggg cgtcccgacg tatgcggcca atccggagtt cgagaaatat 1080

gcgactcttc cgaccgacag tgccaacaag atcatgctgg cggtacattt ctacgatccg 1140

tcggactata ccatcggcga tgcgcagtac agcgactggg ggcataccgg tgcggctgac 1200

aagaaggcct cgggtgggga cgaggaccat gtccgttccg tcttcggcaa cctgtgcacg 1260

aagtatgtgg aaaataacat cccggtctat ctgggcgaat tcggctgctc catgcgggcg 1320

aaatccaata cgcgtgcctg gaaattctat ctgtattaca tggagtatat cgtcaaggca 1380

gccaagacct acggccttcc ttgttatctt tgggacaatg gctccaagag tgagcccggt 1440

aaggaagtcc atggctatat agaccatgga acaggtgact atatcggcaa tagcaaggaa 1500

gtaatcgatg tcatgaagaa agcgtggttt accgagtcgg caggttatac cctccagacg 1560

gtatataatg ctgcgccgaa attgtaa 1587

<210> 2
<211> 1152
<212> DNA
<213> Unknown

<220>
<223> Description of sequence: sequence from genomic DNA library from
      rumen ecosystem, particularly nucleic acid sequence of clones
      pBKRR 1 and 21
; RR01-2 Second enzyme (= RR21)
; CDS; (see Figure 9)

<400> 2
atgctgcgcc gaaattgtaa tcatctgaat atgaaacata tagctttata ttttatgctg 60

ggagccgttg ccctggccgc cccgggctgc ggccggcggg ctgaaagacc cgctacgacc 120

ccgaaggacc aggtgatcgc gcaactgcag gaagccgtga acggcggcaa gattctctat 180

gcccaccagg acgacctggt gtatggccac acctggaaag tagaagatgt ggcgggtgat 240

ccgctggagc gttccgacgt caaggccgtg acaggattct atccggcgat ggtcggcttc 300

gacctgggcg gcatcgagat gggctgggag gccaatctgg acggcgtccc gttcgacctg 360

atgcgccggg cggccgtgac ccacgcctcc cgcggcggca tcgtgacctt ctcctggcat 420

ccgcgcaatc cgttgatcgg cggagacgcc tgggatgtct cttccgacca ggtcgtcgct 480

tccatcctcg aaggagggga gaagcatgac ctgttcatgg aatggctcag gcgtgcgggc 540

gatttcctcg cgtcgctcaa ggatgcggac gggcggccgg tctccttcat cctccgtccc 600

tggcacgagc acatcggcag ctggttctgg tggggcggcc gtctctgctc cgagcagcag 660

tacaaggacc tgttccgcct gacccacgac tacctgaccg tcacccgcgg tttgacggac 720

atcgtctggt gctattcccc gaactccgga atttcgccac agcagtacat gagccggtat 780

ccgggcgacg actgtgtcga tatcctcgga atggacgctt atcagtatgt gggcgatcag 840

ccgctggagg atgcggagac cgctttcgtc gcacaggtcc gggaccacct ggccttcatg 900

aaagacctgg cgcaggagca cgggaaactg atgtgcctgt ccgagaccgg attcgaaggc 960

atccccgatc ctgcctggtg gaccggcacc ctgctcccgg ccatccagga ttttccgatt 1020

gcgtatgtcc tgacctggcg caatgcccac gacaagccgg aacatttcta tgccgcctgg 1080

cccggatcgg gtgacgaagc ggatttcaag gcttttgccg aaaaggataa catcctgttc 1140

ctgaacgagt aa 1152

<210> 3
<211> 1068
<212> DNA
<213> Unknown

<220>
<223> Description of sequence: sequence from genomic DNA library from
      rumen ecosystem, particularly nucleic acid sequence of clones
      pBKRR 2 and 16
; RR02-1 (First enzyme) (= RR16)
; CDS; (see Figure 10)

<400> 3
atggctttga cctcatgcgg taccggaaca aaaaagccca tggaaacgcc caaagaccag 60

ctggttcacc agttgttcac ctatgctgcg aaagggcaga tcgcttacgg ccaccaggac 120

gacctggcct atggccacaa ttgggtggtg accgactggg agaacgaccc gctggagcgc 180

tcggacgtga aggccgttac cggcaagtac cccgccgtcg tggggttcga cctgggcggc 240

atcgagctgg gccacgccaa gaatctggac ggcgtgccgt tcggcctgat gcgaaaggcg 300

gcgcagaagc acgtggagcg tggtggcatt gtcaccttct cctggcatcc gcgcaatccg 360

cttaccggcg gcgatgcctg ggacatcagc tccgaccagg tggtgaaatc ggttctgacc 420

ggcggggaga agcatggcgt ttttatgctt tggctcactc gtgcggccga ttttatcgaa 480

agactggggg ccgatgtccc cgtgattttc cgcccctggc acgagaacct gggaagctgg 540

ttctggtggg gaaagaacct ctgcacggaa caggaatacc aggagcttta ccgcatgacc 600

tggctctatt tcaccaagga gcgtggcctg accaacatcc tgtggtgcta ttcgcccaac 660

ggtccgatag aaccggaact gtatatgtcc cgctatcccg gcgatgaatt cgtggatatc 720

ctggggacgg atatttatga gtatgtcggg gccgacggcc tggaagaagc cggcgtgcgt 780

tttagtatgg aggtgaaaag tatgctgacg gccatgaatg taatggcgac ggatcaccac 840

aaactcatgt gcctgtcgga aaccggcctg gaaggcatcc ccagccccac ttggtggacg 900

ggcgtactgg aacccgccat ccgggaattc cccatcagtt atgtgctcac ctggcgcaac 960

gcccatgata tccctacgca tttctatgcg gcctgggagg gctttgaaca cgctgccgac 1020

atgaaggcgt tcagcgagtt ggacaatatt gtatttttag acgaataa 1068

<210> 4
<211> 1599
<212> DNA

<213>  Unknown

<220>
<223>  Description of sequence: sequence from genomic DNA library from
      rumen ecosystem, particularly nucleic acid sequence of clones
      pBKRR 2 and 16
; RR02-2 (Second enzyme) (= RR16)
; CDS; (see Figure 10)

<400>  4
atgaaaacga tccgttccat agccttttgc accgcggtgc ttgccctgct ttctacagct      60

tgccagaagc ctgatccaac cccgaccccc gtagatgaag cccccacttc catcatgctc     120

agccagagca gtttttctgt ggcaaatacc ggagagaaac tgtccttgac ggtaacggcg     180

ccagccaggc ctgccgtttc cggcctgccg gactggattt ccttgacgga tggaacctat     240

tccaagtaca agattacatt cggtcttatg gtagcggcca atgccggtta tcaggagcgg     300

acggccaccc tgaccgtcac ctcttccggc gcttcttccg tgactgttac cgtgacgcag     360

gccgcttccg cagaaccgga gccgcccacc tccgatccgg acccggataa taaccctgca     420

tggaaaatgg cgatgaacct gggccttgga tggaacatgg gcaaccagtt tgacggctat     480

tacaacggct cctgggcggg agagaaagaa ggatatccag gggagtgtgt ctggcagcct     540

gatgacagcc ataaggccac gcaggctact ttcgaagggc tgaagaaagc cgggttcacc     600

agcgtccgta ttccggtgag ctggcttcgg atgattggtc cggctcctgc ctacaccatc     660

gatgaaacct ggcttggcag ggtgtacgaa gtggtcggat tcgcacacaa tgcagggctt     720

aacgtgattg tgaatacgca tcatgacgag aaccatggag taaacaatac gtaccagtgg     780

ctggatatca agaatgctgc caataattcc gctctgaacc aggccatcaa ggaggaaatc     840

aaggcggtct ggacccagat tgccgaaaag ttcaaagact gcggcgactg gctcatcctg     900

gagagtttca atgagttgaa cgacggtggc tggggctgga gcgatgcttt ccgggccaat     960

ccgtccaagc agtgcgatat cctgaacgag tggaatcagg tcttcgtgga tgccgtccgg    1020

gccaccggcg gagaaaatgc cacgcgctgg ctgggcgttc ccacttatgc ggccaacccg    1080

gaatacacta cttatttcac gatgccctct gatcctgccg gaaagacaat gctggccgtc    1140

catttctacg acccctccga ctataccatc ggcaaggagc agtactccga ctggggacat    1200

accggccagg ccgggcagaa agctacctgg ggcgatgaag accatgtacg ggaagtgttc    1260

ggaaaactga acgccagttt tgtcgaaaag aagattccag tctatctggg cgaattcggc    1320

tgctcgatgc gtcgcaaatc cgacagccgt gcctgggctt tctacaagta ctatctggag    1380

tatgtggtaa aagcggccag gacctatggc ctcccgtgtt tcctgtggga caacggcggc    1440

accgactccg gccaggaaca gcatggatat atccatcatg gtaacggcag ttatctcgga    1500

aacagcaagg agctcgtcga cttgatggtg aaagcctggt ttacagataa ggaaggatat    1560

accctccaga ccatctacaa cagtgctccc aagttctga                           1599

<210>  5
<211>  1548
<212>  DNA
<213>  Unknown

<220>
<223>  Description of sequence: sequence from genomic DNA library from
       rumen ecosystem, particularly nucleic acid sequence of clones
       pBKRR 22, 6, 8 and 23; (see Figure 11)

<400>  5
atgctgttgg catctttctc attgtttagt tgtggcggaa gcgatggaga cgactccacg      60

ccgacaccca gcgtaagcct gcagctagtt tcgcaatcga tagccgaagg tgcccaggtg     120

gatgctgcat cgactactgt ccttacactg aattataaca accctgtcag agtgaccgga     180

aatggtgtta ccctcaatgg aatagcagtg aaagccaagg tttcgggcaa cacatctgta     240

gaaatcccgc ttacgctggt agaaggcacg gtctatacgc tgaaggtggc gtggggtgcc     300

atcgtggctg ccgctgacgg aaaagttgtg gctcctgagt ttacgctgaa cttcactacc     360

aagggaaatc agcaacctcc catcgacgag aacagtccta tcgccaagaa aatgggctgg     420

ggatggaacc tgggcaacca tttcgacacc agcagcggtg ctgacggtgt gcgtccgcag     480

tggggatatt gggacaatgc caaacccaca caggtcctct acaacaacct tagaaacgct     540

ggaaccagca ccgtacgtat cggcgtaaca tggggcaact accagaatac atcgaactgg     600

gacatagatg ctaactacat agcagaagtc aggcagaatg tggagtgggc cgaagctgcc     660

ggactgaacg ttatcctgaa catgcaccac gatgagtact ggctcgacat taagggcgct     720

gccaacaatt cgtcgaccaa caccaatatc aagaaccgta tcgagaaaac ctggaaacag     780

attgccgaag ccttcaagga caagggcgag tttctcatct tcgagtcgtt caacgagatt     840

caggacggcg gctggggatg gggcgcaaac cgtaccgacg gcggtaagca gtataaaacc     900

cttaacgaat ggaaccagtt ggtggtgaac accattcgtg ccaccggcgg caacaacgcc     960

acccggtgga taggcatacc ttcttacgcc gctaatcctt cgctcgcact cgaaaccggc    1020

ttcgtgcttc ctaccgatgc tgccaaccgc ctgatggtca gcgtccactt ctacgacccc    1080

agcaacttca ccctgtcacc ctatgttagc gatgacagca gtgagtataa aagtggtggc    1140

tacagcgagt ggggccacac tgccgctaag ggcaaggccg atactggcag caacgaagac    1200

catgtagtag ctattttcaa gaagttgcac gataagttcg tggctaagaa tattcccgtt    1260

tacatcggcg agtacggttg cgtgatgcac aagaacaccc gttccaacta cttccgcaac    1320

tactatctcg agtatgtatg ccgtgcagcc catgagtatg ggatgccgct ctgcatctgg    1380

gataacaacc aaacgggtgg cggcaacgag catcatggct attttaacca caccgacggt    1440

cagtatctca atgccatgga gtcgcttgtg aagacaatga tcaaggccgc caccagcgat    1500

gatgccagtt acaccctcga gagcgtctat aacaaagctc cgaagtaa 1548

<210> 6
<211> 1995
<212> DNA
<213> Unknown

<220>
<223> Description of sequence: sequence from genomic DNA library from
      rumen ecosystem, particularly nucleic acid sequence of clone
      pBKRR 7
; RR07
; CDS;(see Figure 12)

<400> 6
gtgatcgcag cggtcctatt ggtaggggcc gttttgatag aaaagtacgg taacctggct 60

acatggcaac ttttgctggt ttatcttgtc ccctacttgt acatcggata cgacacactg 120

aaagaggcag cagaaggtat cgcccacggt gatgctttca acgagcactt cttgatgtcg 180

attgccaccc tcggagcttt agccatcggt tttctgccag gtgcagagac acagtttccc 240

gaggccgtct tcgtgatgct cttcttccag gtgggtgagc tgtttgaggg ctacgccgaa 300

gggaagagtc gcgacagcat tgcccatctg atggatatcc gtccggatgt ggctcacctt 360

ctcgacgaag gaggaaggac gaaggaggaa ggagagtcca cccctaaccc ctcccaaagg 420

gagggagatg tgaaggatgt gcgtcctgag aatgtggagg tgggttctat catcctgatc 480

cgccctggtg agaagattcc actagacggt gtggttctgg agggtagttc agctttgaac 540

acggtggcgc tgactggtga gagcatgcca cgcgacatta ctgttggcga tgaggtgatc 600

tcaggatgca tcaacctgtc gggggttatc aaggtgcgta ccaccaagag ttttggtgag 660

agtaccgtat cgaagatcat cagtctggta gaacatgcca gcgagcacaa gtcgaagagc 720

gaagcattca tcagtaagtt cgcccgtatc tacaccccga tagtggtctt tgccgccctc 780

gccctggcca ttctcccccc actattggct gccatcacca tttccccatt taattttcaa 840

ttgtcaattt tcaattctca attttctacc tggctctacc gtgccctgat gttcttggtg 900

gtgtcttgtc cttgcgcact ggtcatcagc gttcccctca cctttttcgg cggtatcggc 960

ggtgcgtcac gcaaaggtat cctcatcaag ggcgccaact acatggatat cctggccaag 1020

gtgaagaccg ttgtctttga taagaccggt accctcaccc acggacaatt tgccgtagaa 1080

gctgtgcatc ctaatcaagg ggagtcatca tgtagtaatt ctgcagcagc agactctcac 1140

ctctcatctc tcatctctca tctccttcat ctggccgccc atgtggaaag tttctcctcc 1200

caccccattg ctgccgccct gcgtgatgct tttcctgagg cagctcacga cgattgcgag 1260

gtgagcgata tcaaggaaat cgctggtcat ggcattcagg cccgggtggg caatgacatc 1320

gtttgtgtgg gtaacacgaa gatgatggac agcttgggtg ttgcctggca cgactgtcat 1380

catcctggaa ccatcatcca tgtggccatc aacggggaat atgccggaca tatcatcatc 1440

aacgacatga tcaaggacga cagtgccgaa gccatccgac aactgaagga gttgggagtg    1500

gaaagaaccg ttatgcttac gggcgaccgt aaagaggtag ccgaccatgt ggctaagatg    1560

ttggatatca ctgaatacca tgccgaactc ctccccacag acaaagtttc tttcgttgag    1620

caagagctgt ctgctcactc ttcttccacg tctcacccct catatgtggc ctttgtcggc    1680

gacggtatca atgatgctcc tgtactggct cgagctgatg tgggtatcgc catgggcgga    1740

ttgggtagtg atgctgccat tgaagcagcc gacgtggttc tgatggacga tcagccctcg    1800

aagattgccc aggctatccg cattgcccgt cgcactcttt ccatcgcccg acagaacgtg    1860

tggttcgcca tcggtgtaaa aatcgccgta ctcatcctcg ccaccttcgg cctcagcacc    1920

atgtggatgg ccgtctttgc cgatgtgggc gtcaccgtac tggccgtact caacgccatg    1980

cgaaccatgt attaa                                                    1995

<210>  7
<211>  1062
<212>  DNA
<213>  Unknown

<220>
<223>  Description of sequence: sequence from genomic DNA library from
       rumen ecosystem, particularly nucleic acid sequence of clone;
       pBKRR 9
; RR09
; CDS;(see Figure 13)

<400>  7
atgaaattag tagagtttaa cgcagaagaa aactatatag aagatttttt aagtttacct      60

aaaaaattat atacaaaatt agataatatg gaagattcta acactatgaa agatattctt     120

actaataatc atcctttaag taacgatttt aaattaaata aatatttagt atataaagat     180

gatgaagtag tcgcaaggtt tataataact gaatacaatg atgataataa agtgtgttat     240

ataggatttt ttgaatgtat taatgataaa aaagtagcta agttcttatt tgatgaagct     300

cacaaaatag ctaaagaaaa gaaatataaa aagattattg gtccagtaga tgcatccttt     360

tggattaaat accgtctaaa aataaataaa tttgaaagac cctataccgg agaaccatat     420

aataaagatt attatttaaa gttatttaca gataataaat ataaaatatg tgatcattat     480

acttcacaaa gctatgatat agtagatgat agttatttta atgataaatt tactgaacac     540

tataatgaat ttaaaaaact aggttatgaa ataattaaac ctaaaccaga agattttgat     600

aaatgtatgt cagaagtata tgatttaatt actaagttat atagcgattt tcctatattt     660

aaaaatttaa aaaaagaatc tttcttagaa atatataaat cttataaaaa aattattaat     720

atgaatatgg ttagaatggc atattttaaa ggacaagcag taggtttcta tataagtgta     780

cctaactata ataatattgt atatcatatt aatcccataa atatattaaa gattcttcat     840

caaagaaaac acccaaaagg ctatgtaatg ctttatatgg gagtagattc aaatcataga 900

ggattaggta aagcattagt atattcaata gtagaagaat taaagaaaaa taatctacca 960

agtattggag ctttagctca tgatggtaaa attagtcaga attatgcaaa agaaaaaata 1020

aattctagat atgaatatgt attacttgaa aggagtattt aa 1062

<210> 8
<211> 1611
<212> DNA
<213> Unknown

<220>
<223> Description of sequence: sequence from genomic DNA library from
      rumen ecosystem, particularly nucleic acid sequence of clones
      pBKRR 11, 10 and 12
; RR10=RR11=RR12
; CDS;(see Figure 14)

<400> 8
atgagaattt taaaattatt ttttattgcg cttataacag ctgctccaat cttcagttat 60

gctcagacag tcgatgatat gaaatcagtc tttgttgatg cccacacgtg gacagcctct 120

ataaaaatgg gttggaattt gggtaatgcc cttgaatgtc agacaggatg gaatgataag 180

gcgttttgtt ggaatcctgc aaacaacttt aatgcagaga cctcgtgggg aaatcccaaa 240

accacaaaag aaatgcttca ggctgttcgt gaggcaggtt ttgatgctgt gcgtattcct 300

gtaaactggg gtgcccatat aagcgatgag tcaacttgta ctattgatgc agcttggatg 360

aatcgtgttc aagaagttgt tgactgggct ctgcaacttg gtttcaaggt attgctcaat 420

acccatcatg agtattggct tgagtggcat ccaacctatg accgtgaaca ggctaataat 480

aataaacttg ctaagatatg gatgcaggtt gctaatagat ttaaggatta tggtccagac 540

cttgcatttg ccggtatcaa cgaggttcat ttggatggta agtggaatgt tccaacagaa 600

gagaatactg cagttaccaa tagttataat cagacatttg ttaataccgt tcgtgcaaca 660

ggtggcaata acttatatcg taatcttgta gtacaatgtt attcatgtaa tccagattac 720

ggcttgacag gttttgttgt tcctgctgac aaggttgaga atcgtcttag catagaatat 780

cattattatc gtccatggga ttatgccggc gatgctccaa agtattacta ttggggtgag 840

gcgtataagc aatatggcga ggtgagcaca gccgacaacg aggcaaaggt taatgccgat 900

tttgcacgtt acaaggcagc ttggtatgac aagggcttgg gtgtgattat gggtgaatgg 960

ggtgcgagtc agcattatca ggtagagagc aaggcaaaac agcttgaaaa cctatattac 1020

catttcaaga cagttcgtga ggcagcacag aaaaataata ttgctacttt tgtatgggat 1080

aataatgcat gtggaaatgg tggtgacaag tttggaattt ttgatagaaa tgacaatatg 1140

tctgtagcgt tcccagaagc actaaatgct attcaggagg catcgggaca aacggttgtt 1200

gactatagca gacaggcttt gcagccttct gtggtttatc agggtgatga gctaatgaat 1260

tggggtgaag gcaagcagtt gattattgct ggaagtaagt ttgcttattt taccgcagag 1320

agcaaactta tggttactct tgatgctgaa ccaggtgcag attatgatat gttgcagttt 1380

gcttatggcg actggaaatc aaagccattg atgattattt cgggcaggag ttacaaggga 1440

caggttgagc cttcaaagat taatggttct cgcaatacat ataccctgtt tattggtttt 1500

aaggaatcgt ctcttaacca actgaaggat aagggaataa cccttcaggg gcatggtctt 1560

cgtttgcgta atgtagttgt gatgccagaa gggcttgtat tagggttata a 1611

<210> 9
<211> 963
<212> DNA
<213> Unknown

<220>
<223> Description of sequence: sequence from genomic DNA library from
      rumen ecosystem, particularly nucleic acid sequence of clone
      pBKRR 13
; RR 13
; CDS;(see Figure 15)

<400> 9
atgacaatga agaaactact gaccatcatt atgttgagtt tgttggcatg tcacgtacac 60

gccaacgacc ccgtcaagca gtacggaaaa ctgcaagtaa aaggtgccca gctctgcgac 120

caaaagggca atcctgtcat tctgcgtggt gtcagtctgg gctggcacaa cctgtggccg 180

cgcttctata acaagggggc cgtggagacg ctgaagaacg actggcactg tagtgtcatc 240

cgtgcggcta tcggacagca tatcgaggac aacttccagg agaaccccga gttcgccatg 300

cagtgtctga cgcccgtcgt tgaagccgcc atcaagcaga acgtctacgt catcatcgac 360

tggcactcgc acaaactgat gacggaagag gccaagcagt tctttggcga gatggcccgg 420

aaatacggca agtatccgca catcatctac gaaatctaca acgagcccat cgcggacacg 480

tggaccgatc tgaagaaata cgctcaggag gttattggag aaatccgcaa atacgataag 540

gacaatgtcg tactcgtggg ttgcccccac tgggatcagg atatccatct ggttgccgag 600

agtccgttgg agggcctctc gaacgtcatg tacaccgtgc atttctatgc cgctacgcat 660

ggtgaagacc tgcgtcagcg tacagaagaa gctgccaaac gaggtattcc catcttcatt 720

tccgaaagcg gtgccaccga ggccagcggc gacggtaaga ttgatgagga gagcgaggag 780

gaatggatca agatgtgcga acgcctgggc atcagctggc tgtgctggag catcagcgac 840

aagaacgagt cgagttcgat gctgctgcct cgtgccactg ctacaggtcc ttggcccgat 900

gacgtcatca agaaatacgg caaactggtg aagggactgc tggagaaata taatgcgagg 960

taa 963

<210> 10

<211>  1452
<212>  DNA
<213>  Unknown

<220>
<223>  Description of sequence: sequence from genomic DNA library from
       rumen ecosystem, particularly nucleic acid sequence of clones
       pBKRR 14 and 20-1
; RR14=RR20-1
; CDS;(see Figure 16)

<400>  10
atgatcaatg tcctgcagca tcatcccgag gtggagctgt cggtggacaa aacttccgtc      60

tccttcaacc gttccggggg agaggagacc ttcacggtca cttcttccac ccagcctcat     120

gtgtcggccg acgtttcctg ggttgtggtc gagacgggca agattgacaa ggatcaccat     180

accgaagtca gggtcttggc gggggcgaac cggaaggagg cttccgcagg aaccttgacg     240

gtgtcctgca gcgacaagaa agtgtcggtc agcgtcaagc aggaggcatt tgtcgctcct     300

tccgtcgcca gcacgacggc ggtgactccg cagatggtgt tcgatgccat gggaccgggc     360

tggaacatgg gcaaccacat ggacgccatc agcaacggcg tgtccggcga gacggtctgg     420

ggcaatccca aatgcaccca ggccacgatg gacggggtca aagcggccgg ttacaaggcc     480

gtccgcatct gcacgacctg ggaaggccat atcggcgccg ctccggcgta tgcgctcgag     540

cagaaatggc tcgaccgcgt ggcggagatt gtcgggtatg ccgaaaaagc cggcctcgtc     600

gccatcgtga acacgcatca tgacgagagc tattggcagg acatcagcaa gtgctataac     660

aacgcggcga atcacgagaa ggtcaaggac gaggtgttca gcgtctggac ccagatcgcg     720

gagaagttca aggacaaggg cgaatggctc gtgttcgagt ccttcaacga gatccaggac     780

ggcggctggg gctggagcga cgccttccgg aagaatccgg acgcgcagta caaggtgctc     840

aatgaatgga accagacctt cgtggacgcg gtccgcagca cgggcgggca gaatgcgacc     900

cgctggctcg gaattccggg ctacgcctgc aacccgggct tcacgatcgc cggtctggtg     960

ctgcccaagg attacactac cgccaaccgc ctgatggtgg ccgtccatga ctacgacccg    1020

tacgactata cgctcaagga tccgctcatc cgccagtggg ggcatacggc cgatgcggac    1080

aagcgcccga gcggggacaa cgagaaggct gtcgtggatg tcttcaacaa tctcaaggcc    1140

gcctacctgg acaagggtat tccggtctat ctcggcgaga tgggctgctc acgccatacc    1200

gccgcggact tcccgtatca gaagtattac atggagtatt tctgcaaggc cgccgccgac    1260

cgcctgctgc cgatgtacct ctgggacaac ggcgccaagg gtgtcggctc ggagcgccac    1320

gcctacatcg accacggaac cggacagttc gtggacgagg atgcccggac gcttgtgggg    1380

ctgatggtga aggccgtgac gacgaaagat gcgtcctaca cgctggagag cgtctataac    1440

tccgcgccgt aa                                                        1452

<210>  11
<211>  528
<212>  PRT
<213>  Unknown

<220>
<223>  Description of sequence: sequence derived from genomic DNA library
       from rumen ecosystem, particularly amino acid sequence of clones
       pBKRR 1 and 21
; RR01-1 First enzyme (= RR21) ;(see Figure 17)

<400>  11

Met Lys Leu Tyr Trp Lys Tyr Leu Ser Leu Thr Ala Val Leu Ala Val
1               5                   10                  15

Leu Ala Ser Cys Gly Gly Asn Ala Asp Pro Asp Pro Glu Pro Thr Pro
            20                  25                  30

Ser Ala Pro Ser Ser Ile Thr Leu Ser Val Asn Ser Phe Thr Val Ala
        35                  40                  45

Gln Ala Gly Glu Thr Leu Ser Leu Asp Ile Thr Ala Pro Thr Arg Pro
    50                  55                  60

Lys Leu Ala Leu Pro Asn Trp Ile Thr Leu Lys Asp Gly Thr Tyr Gln
65                  70                  75                  80

Asn Tyr Lys Ile Thr Val Gly Leu Thr Val Ser Ala Asn Asp Thr Tyr
                85                  90                  95

Gln Ala Arg Glu Ala Thr Val Thr Val Ser Ala Thr Gly Thr Phe Asp
            100                 105                 110

Val Thr Phe Lys Val Ser Gln Ala Gly Lys Glu Pro Glu Pro Thr Pro
        115                 120                 125

Pro Asp Pro Ser Gly Gly Asp Asn Asp Ala Trp Gln Met Ala Lys Lys
    130                 135                 140

Leu Gly Leu Gly Trp Asn Met Gly Asn His Phe Asp Ala Phe Tyr Asn
145                 150                 155                 160

Gly Ser Trp Ala Gly Glu Lys Glu Gly Tyr Pro Asp Glu Glu Val Trp
                165                 170                 175

Gly Ala Ser Lys Ala Thr Gln Thr Thr Phe Asn Gly Val Lys Asn Ala
            180                 185                 190

Gly Phe Thr Ser Val Arg Ile Pro Val Ser Trp Leu Lys Met Ile Gly
        195                 200                 205

Pro Ala Pro Glu Tyr Lys Ile Asp Glu Thr Trp Leu Asn Arg Val Tyr
    210                 215                 220

Glu Val Ala Gln Tyr Ala His Asn Ala Gly Leu Thr Ala Ile Val Asn
225                 230                 235                 240

Thr His His Asp Glu Asn His Gly Val Asp Asn Thr Tyr Gln Trp Leu
                245                 250                 255

Asp Ile Lys Asn Ala Ser Thr Asp Ala Ser Val Asn Thr Arg Ile Lys
            260                 265                 270

Glu Glu Ile Lys Ala Val Trp Thr Gln Ile Ala Asn Lys Phe Lys Asp
        275                 280                 285

Cys Gly Asp Trp Leu Ile Leu Glu Ser Phe Asn Glu Leu Asn Asp Gly
    290                 295                 300

Gly Trp Gly Trp Ser Asp Ala Phe Arg Ala Asn Pro Thr Lys Gln Cys
305                 310                 315                 320

Asn Ile Leu Asn Glu Trp Asn Gln Val Phe Val Asp Ala Val Arg Ala
                325                 330                 335

Thr Gly Gly Asn Asn Ala Thr Arg Trp Leu Gly Val Pro Thr Tyr Ala
            340                 345                 350

Ala Asn Pro Glu Phe Glu Lys Tyr Ala Thr Leu Pro Thr Asp Ser Ala
        355                 360                 365

Asn Lys Ile Met Leu Ala Val His Phe Tyr Asp Pro Ser Asp Tyr Thr
    370                 375                 380

Ile Gly Asp Ala Gln Tyr Ser Asp Trp Gly His Thr Gly Ala Ala Asp
385                 390                 395                 400

Lys Lys Ala Ser Gly Gly Asp Glu Asp His Val Arg Ser Val Phe Gly
                405                 410                 415

Asn Leu Cys Thr Lys Tyr Val Glu Asn Asn Ile Pro Val Tyr Leu Gly
            420                 425                 430

Glu Phe Gly Cys Ser Met Arg Ala Lys Ser Asn Thr Arg Ala Trp Lys
        435                 440                 445

Phe Tyr Leu Tyr Tyr Met Glu Tyr Ile Val Lys Ala Ala Lys Thr Tyr
    450                 455                 460

Gly Leu Pro Cys Tyr Leu Trp Asp Asn Gly Ser Lys Ser Glu Pro Gly
465                 470                 475                 480

Lys Glu Val His Gly Tyr Ile Asp His Gly Thr Gly Asp Tyr Ile Gly
                485                 490                 495

Asn Ser Lys Glu Val Ile Asp Val Met Lys Lys Ala Trp Phe Thr Glu
            500                 505                 510

Ser Ala Gly Tyr Thr Leu Gln Thr Val Tyr Asn Ala Ala Pro Lys Leu
        515                 520                 525

<210>  12
<211>  383
<212>  PRT
<213>  Unknown

<220>
<223>  Description of sequence: sequence derived from genomic DNA library
       from rumen ecosystem, particularly amino acid sequence of clones
       pBKRR 1 and 21
; RR01-2 Second enzyme (= RR21) ;(see Figure 17)

<400>  12

Met Leu Arg Arg Asn Cys Asn His Leu Asn Met Lys His Ile Ala Leu
1               5                   10                  15

Tyr Phe Met Leu Gly Ala Val Ala Leu Ala Ala Pro Gly Cys Gly Arg
            20                  25                  30

Arg Ala Glu Arg Pro Ala Thr Thr Pro Lys Asp Gln Val Ile Ala Gln
        35                  40                  45

Leu Gln Glu Ala Val Asn Gly Gly Lys Ile Leu Tyr Ala His Gln Asp
    50                  55                  60

Asp Leu Val Tyr Gly His Thr Trp Lys Val Glu Asp Val Ala Gly Asp
65                  70                  75                  80

Pro Leu Glu Arg Ser Asp Val Lys Ala Val Thr Gly Phe Tyr Pro Ala
                85                  90                  95

Met Val Gly Phe Asp Leu Gly Gly Ile Glu Met Gly Trp Glu Ala Asn
            100                 105                 110

Leu Asp Gly Val Pro Phe Asp Leu Met Arg Arg Ala Ala Val Thr His

115                 120                 125

Ala Ser Arg Gly Gly Ile Val Thr Phe Ser Trp His Pro Arg Asn Pro
    130                 135                 140

Leu Ile Gly Gly Asp Ala Trp Asp Val Ser Ser Asp Gln Val Val Ala
145                 150                 155                 160

Ser Ile Leu Glu Gly Gly Glu Lys His Asp Leu Phe Met Glu Trp Leu
                165                 170                 175

Arg Arg Ala Gly Asp Phe Leu Ala Ser Leu Lys Asp Ala Asp Gly Arg
            180                 185                 190

Pro Val Ser Phe Ile Leu Arg Pro Trp His Glu His Ile Gly Ser Trp
        195                 200                 205

Phe Trp Trp Gly Gly Arg Leu Cys Ser Glu Gln Gln Tyr Lys Asp Leu
    210                 215                 220

Phe Arg Leu Thr His Asp Tyr Leu Thr Val Thr Arg Gly Leu Thr Asp
225                 230                 235                 240

Ile Val Trp Cys Tyr Ser Pro Asn Ser Gly Ile Ser Pro Gln Gln Tyr
                245                 250                 255

Met Ser Arg Tyr Pro Gly Asp Asp Cys Val Asp Ile Leu Gly Met Asp
            260                 265                 270

Ala Tyr Gln Tyr Val Gly Asp Gln Pro Leu Glu Asp Ala Glu Thr Ala
        275                 280                 285

Phe Val Ala Gln Val Arg Asp His Leu Ala Phe Met Lys Asp Leu Ala
    290                 295                 300

Gln Glu His Gly Lys Leu Met Cys Leu Ser Glu Thr Gly Phe Glu Gly
305                 310                 315                 320

Ile Pro Asp Pro Ala Trp Trp Thr Gly Thr Leu Leu Pro Ala Ile Gln
                325                 330                 335

Asp Phe Pro Ile Ala Tyr Val Leu Thr Trp Arg Asn Ala His Asp Lys
            340                 345                 350

Pro Glu His Phe Tyr Ala Ala Trp Pro Gly Ser Gly Asp Glu Ala Asp
        355                 360                 365

Phe Lys Ala Phe Ala Glu Lys Asp Asn Ile Leu Phe Leu Asn Glu
    370                 375                 380

<210>  13
<211>  332
<212>  PRT
<213>  Unknown

<220>
<223>  Description of sequence: sequence derived from genomic DNA library
       from rumen ecosystem, particularly amino acid sequence of clones
       pBKRR 2 and 16
; RR02-1 (First enzyme) (= RR16) ;(see Figure 18)

<400>  13

Met Ala Leu Thr Ser Cys Gly Thr Gly Thr Lys Lys Pro Met Glu Thr
1               5                   10                  15

Pro Lys Asp Gln Leu Val His Gln Leu Phe Thr Tyr Ala Ala Lys Gly
            20                  25                  30

Gln Ile Ala Tyr Gly His Gln Asp Asp Leu Ala Tyr Gly His Asn Trp
        35                  40                  45

Val Val Thr Asp Trp Glu Asn Asp Pro Leu Glu Arg Ser Asp Val Lys
    50                  55                  60

Ala Val Thr Gly Lys Tyr Pro Ala Val Val Gly Phe Asp Leu Gly Gly
65                  70                  75                  80

Ile Glu Leu Gly His Ala Lys Asn Leu Asp Gly Val Pro Phe Gly Leu
                85                  90                  95

Met Arg Lys Ala Ala Gln Lys His Val Glu Arg Gly Gly Ile Val Thr
            100                 105                 110

Phe Ser Trp His Pro Arg Asn Pro Leu Thr Gly Gly Asp Ala Trp Asp
        115                 120                 125

Ile Ser Ser Asp Gln Val Val Lys Ser Val Leu Thr Gly Gly Glu Lys
    130                 135                 140

His Gly Val Phe Met Leu Trp Leu Thr Arg Ala Ala Asp Phe Ile Glu
145                 150                 155                 160

Arg Leu Gly Ala Asp Val Pro Val Ile Phe Arg Pro Trp His Glu Asn
                165                 170                 175

Leu Gly Ser Trp Phe Trp Trp Gly Lys Asn Leu Cys Thr Glu Gln Glu
            180                 185                 190

Tyr Gln Glu Leu Tyr Arg Met Thr Trp Leu Tyr Phe Thr Lys Glu Arg
        195                 200                 205

Gly Leu Thr Asn Ile Leu Trp Cys Tyr Ser Pro Asn Gly Pro Ile Glu
    210                 215                 220

Pro Glu Leu Tyr Met Ser Arg Tyr Pro Gly Asp Glu Phe Val Asp Ile
225                 230                 235                 240

Leu Gly Thr Asp Ile Tyr Glu Tyr Val Gly Ala Asp Gly Leu Glu Glu
                245                 250                 255

Ala Gly Val Arg Phe Ser Met Glu Val Lys Ser Met Leu Thr Ala Met
            260                 265                 270

Asn Val Met Ala Thr Asp His His Lys Leu Met Cys Leu Ser Glu Thr
        275                 280                 285

Gly Leu Glu Gly Ile Pro Ser Pro Thr Trp Trp Thr Gly Val Leu Glu
    290                 295                 300

Pro Ala Ile Arg Glu Phe Pro Ile Ser Tyr Val Leu Thr Trp Arg Asn
305                 310                 315                 320

Ala His Asp Ile Pro Thr His Phe Tyr Ala Ala Trp
                325                 330

<210>  14
<211>  532
<212>  PRT
<213>  Unknown

<220>
<223>  Description of sequence: sequence derived from genomic DNA library
       from rumen ecosystem, particularly amino acid sequence of clones
       pBKRR 2 and 16
; RR02-2 (Second enzyme) (= RR16) ;(see Figure 18)

<400>  14

Met Lys Thr Ile Arg Ser Ile Ala Phe Cys Thr Ala Val Leu Ala Leu
1               5                   10                  15

Leu Ser Thr Ala Cys Gln Lys Pro Asp Pro Thr Pro Thr Pro Val Asp
            20                  25                  30

Glu Ala Pro Thr Ser Ile Met Leu Ser Gln Ser Ser Phe Ser Val Ala
        35                  40                  45

Asn Thr Gly Glu Lys Leu Ser Leu Thr Val Thr Ala Pro Ala Arg Pro
    50                  55                  60

Ala Val Ser Gly Leu Pro Asp Trp Ile Ser Leu Thr Asp Gly Thr Tyr
65                  70                  75                  80

Ser Lys Tyr Lys Ile Thr Phe Gly Leu Met Val Ala Ala Asn Ala Gly
                85                  90                  95

Tyr Gln Glu Arg Thr Ala Thr Leu Thr Val Thr Ser Ser Gly Ala Ser
            100                 105                 110

Ser Val Thr Val Thr Val Thr Gln Ala Ala Ser Ala Glu Pro Glu Pro
        115                 120                 125

Pro Thr Ser Asp Pro Asp Pro Asp Asn Asn Pro Ala Trp Lys Met Ala
    130                 135                 140

Met Asn Leu Gly Leu Gly Trp Asn Met Gly Asn Gln Phe Asp Gly Tyr
145                 150                 155                 160

Tyr Asn Gly Ser Trp Ala Gly Glu Lys Glu Gly Tyr Pro Gly Glu Cys
                165                 170                 175

Val Trp Gln Pro Asp Asp Ser His Lys Ala Thr Gln Ala Thr Phe Glu
            180                 185                 190

Gly Leu Lys Lys Ala Gly Phe Thr Ser Val Arg Ile Pro Val Ser Trp
        195                 200                 205

Leu Arg Met Ile Gly Pro Ala Pro Ala Tyr Thr Ile Asp Glu Thr Trp
    210                 215                 220

Leu Gly Arg Val Tyr Glu Val Val Gly Phe Ala His Asn Ala Gly Leu
225                 230                 235                 240

Asn Val Ile Val Asn Thr His His Asp Glu Asn His Gly Val Asn Asn
                245                 250                 255

Thr Tyr Gln Trp Leu Asp Ile Lys Asn Ala Ala Asn Asn Ser Ala Leu
            260                 265                 270

Asn Gln Ala Ile Lys Glu Glu Ile Lys Ala Val Trp Thr Gln Ile Ala
        275                 280                 285

Glu Lys Phe Lys Asp Cys Gly Asp Trp Leu Ile Leu Glu Ser Phe Asn
    290                 295                 300

Glu Leu Asn Asp Gly Gly Trp Gly Trp Ser Asp Ala Phe Arg Ala Asn
305                 310                 315                 320

Pro Ser Lys Gln Cys Asp Ile Leu Asn Glu Trp Asn Gln Val Phe Val
                325                 330                 335

Asp Ala Val Arg Ala Thr Gly Gly Glu Asn Ala Thr Arg Trp Leu Gly
            340                 345                 350

Val Pro Thr Tyr Ala Ala Asn Pro Glu Tyr Thr Thr Tyr Phe Thr Met
        355                 360                 365

Pro Ser Asp Pro Ala Gly Lys Thr Met Leu Ala Val His Phe Tyr Asp
    370                 375                 380

Pro Ser Asp Tyr Thr Ile Gly Lys Glu Gln Tyr Ser Asp Trp Gly His
385                 390                 395                 400

Thr Gly Gln Ala Gly Gln Lys Ala Thr Trp Gly Asp Glu Asp His Val
                405                 410                 415

Arg Glu Val Phe Gly Lys Leu Asn Ala Ser Phe Val Glu Lys Lys Ile
            420                 425                 430

Pro Val Tyr Leu Gly Glu Phe Gly Cys Ser Met Arg Arg Lys Ser Asp
        435                 440                 445

Ser Arg Ala Trp Ala Phe Tyr Lys Tyr Tyr Leu Glu Tyr Val Val Lys
    450                 455                 460

Ala Ala Arg Thr Tyr Gly Leu Pro Cys Phe Leu Trp Asp Asn Gly Gly
465                 470                 475                 480

Thr Asp Ser Gly Gln Glu Gln His Gly Tyr Ile His His Gly Asn Gly
                485                 490                 495

Ser Tyr Leu Gly Asn Ser Lys Glu Leu Val Asp Leu Met Val Lys Ala
            500                 505                 510

Trp Phe Thr Asp Lys Glu Gly Tyr Thr Leu Gln Thr Ile Tyr Asn Ser
        515                 520                 525

Ala Pro Lys Phe
    530

<210>  15
<211>  515

<212>  PRT
<213>  Unknown

<220>
<223>  Description of sequence: sequence derived from genomic DNA library
      from rumen ecosystem, particularly amino acid sequence of clones
      pBKRR 22, 6, 8 and 23;(see Figure 19)

<400>  15

Met Leu Leu Ala Ser Phe Ser Leu Phe Ser Cys Gly Gly Ser Asp Gly
1               5                   10                  15

Asp Asp Ser Thr Pro Thr Pro Ser Val Ser Leu Gln Leu Val Ser Gln
            20                  25                  30

Ser Ile Ala Glu Gly Ala Gln Val Asp Ala Ala Ser Thr Thr Val Leu
        35                  40                  45

Thr Leu Asn Tyr Asn Asn Pro Val Arg Val Thr Gly Asn Gly Val Thr
    50                  55                  60

Leu Asn Gly Ile Ala Val Lys Ala Lys Val Ser Gly Asn Thr Ser Val
65                  70                  75                  80

Glu Ile Pro Leu Thr Leu Val Glu Gly Thr Val Tyr Thr Leu Lys Val
                85                  90                  95

Ala Trp Gly Ala Ile Val Ala Ala Ala Asp Gly Lys Val Val Ala Pro
            100                 105                 110

Glu Phe Thr Leu Asn Phe Thr Thr Lys Gly Asn Gln Gln Pro Pro Ile
        115                 120                 125

Asp Glu Asn Ser Pro Ile Ala Lys Lys Met Gly Trp Gly Trp Asn Leu
    130                 135                 140

Gly Asn His Phe Asp Thr Ser Ser Gly Ala Asp Gly Val Arg Pro Gln
145                 150                 155                 160

Trp Gly Tyr Trp Asp Asn Ala Lys Pro Thr Gln Val Leu Tyr Asn Asn
                165                 170                 175

Leu Arg Asn Ala Gly Thr Ser Thr Val Arg Ile Gly Val Thr Trp Gly
            180                 185                 190

Asn Tyr Gln Asn Thr Ser Asn Trp Asp Ile Asp Ala Asn Tyr Ile Ala
        195                 200                 205

Glu Val Arg Gln Asn Val Glu Trp Ala Glu Ala Ala Gly Leu Asn Val

210                 215                 220

Ile Leu Asn Met His His Asp Glu Tyr Trp Leu Asp Ile Lys Gly Ala
225                 230                 235                 240

Ala Asn Asn Ser Ser Thr Asn Thr Asn Ile Lys Asn Arg Ile Glu Lys
                245                 250                 255

Thr Trp Lys Gln Ile Ala Glu Ala Phe Lys Asp Lys Gly Glu Phe Leu
            260                 265                 270

Ile Phe Glu Ser Phe Asn Glu Ile Gln Asp Gly Gly Trp Gly Trp Gly
        275                 280                 285

Ala Asn Arg Thr Asp Gly Gly Lys Gln Tyr Lys Thr Leu Asn Glu Trp
    290                 295                 300

Asn Gln Leu Val Val Asn Thr Ile Arg Ala Thr Gly Gly Asn Asn Ala
305                 310                 315                 320

Thr Arg Trp Ile Gly Ile Pro Ser Tyr Ala Ala Asn Pro Ser Leu Ala
                325                 330                 335

Leu Glu Thr Gly Phe Val Leu Pro Thr Asp Ala Ala Asn Arg Leu Met
            340                 345                 350

Val Ser Val His Phe Tyr Asp Pro Ser Asn Phe Thr Leu Ser Pro Tyr
        355                 360                 365

Val Ser Asp Asp Ser Ser Glu Tyr Lys Ser Gly Gly Tyr Ser Glu Trp
    370                 375                 380

Gly His Thr Ala Ala Lys Gly Lys Ala Asp Thr Gly Ser Asn Glu Asp
385                 390                 395                 400

His Val Val Ala Ile Phe Lys Lys Leu His Asp Lys Phe Val Ala Lys
                405                 410                 415

Asn Ile Pro Val Tyr Ile Gly Glu Tyr Gly Cys Val Met His Lys Asn
            420                 425                 430

Thr Arg Ser Asn Tyr Phe Arg Asn Tyr Tyr Leu Glu Tyr Val Cys Arg
        435                 440                 445

Ala Ala His Glu Tyr Gly Met Pro Leu Cys Ile Trp Asp Asn Asn Gln
    450                 455                 460

Thr Gly Gly Gly Asn Glu His His Gly Tyr Phe Asn His Thr Asp Gly
465                 470                 475                 480

Gln Tyr Leu Asn Ala Met Glu Ser Leu Val Lys Thr Met Ile Lys Ala
                485                 490                 495

Ala Thr Ser Asp Asp Ala Ser Tyr Thr Leu Glu Ser Val Tyr Asn Lys
            500                 505                 510

Ala Pro Lys
        515

<210>  16
<211>  664
<212>  PRT
<213>  Unknown

<220>
<223>  Description of sequence: sequence derived from genomic DNA library
       from rumen ecosystem, particularly amino acid sequence of the
       polypeptide and the mature protein of clone pBKRR 7; Polypeptide
       ;(see Figure 20)

<400>  16

Val Ile Ala Ala Val Leu Leu Val Gly Ala Val Leu Ile Glu Lys Tyr
1               5                   10                  15

Gly Asn Leu Ala Thr Trp Gln Leu Leu Leu Val Tyr Leu Val Pro Tyr
            20                  25                  30

Leu Tyr Ile Gly Tyr Asp Thr Leu Lys Glu Ala Ala Glu Gly Ile Ala
        35                  40                  45

His Gly Asp Ala Phe Asn Glu His Phe Leu Met Ser Ile Ala Thr Leu
    50                  55                  60

Gly Ala Leu Ala Ile Gly Phe Leu Pro Gly Ala Glu Thr Gln Phe Pro
65                  70                  75                  80

Glu Ala Val Phe Val Met Leu Phe Phe Gln Val Gly Glu Leu Phe Glu
                85                  90                  95

Gly Tyr Ala Glu Gly Lys Ser Arg Asp Ser Ile Ala His Leu Met Asp
            100                 105                 110

Ile Arg Pro Asp Val Ala His Leu Leu Asp Glu Gly Gly Arg Thr Lys
        115                 120                 125

Glu Glu Gly Glu Ser Thr Pro Asn Pro Ser Gln Arg Glu Gly Asp Val
    130                 135                 140

Lys Asp Val Arg Pro Glu Asn Val Glu Val Gly Ser Ile Ile Leu Ile
145                 150                 155                 160

Arg Pro Gly Glu Lys Ile Pro Leu Asp Gly Val Val Leu Glu Gly Ser
                165                 170                 175

Ser Ala Leu Asn Thr Val Ala Leu Thr Gly Glu Ser Met Pro Arg Asp
            180                 185                 190

Ile Thr Val Gly Asp Glu Val Ile Ser Gly Cys Ile Asn Leu Ser Gly
        195                 200                 205

Val Ile Lys Val Arg Thr Thr Lys Ser Phe Gly Glu Ser Thr Val Ser
    210                 215                 220

Lys Ile Ile Ser Leu Val Glu His Ala Ser Glu His Lys Ser Lys Ser
225                 230                 235                 240

Glu Ala Phe Ile Ser Lys Phe Ala Arg Ile Tyr Thr Pro Ile Val Val
                245                 250                 255

Phe Ala Ala Leu Ala Leu Ala Ile Leu Pro Pro Leu Leu Ala Ala Ile
            260                 265                 270

Thr Ile Ser Pro Phe Asn Phe Gln Leu Ser Ile Phe Asn Ser Gln Phe
        275                 280                 285

Ser Thr Trp Leu Tyr Arg Ala Leu Met Phe Leu Val Val Ser Cys Pro
    290                 295                 300

Cys Ala Leu Val Ile Ser Val Pro Leu Thr Phe Phe Gly Gly Ile Gly
305                 310                 315                 320

Gly Ala Ser Arg Lys Gly Ile Leu Ile Lys Gly Ala Asn Tyr Met Asp
                325                 330                 335

Ile Leu Ala Lys Val Lys Thr Val Val Phe Asp Lys Thr Gly Thr Leu
            340                 345                 350

Thr His Gly Gln Phe Ala Val Glu Ala Val His Pro Asn Gln Gly Glu
        355                 360                 365

Ser Ser Cys Ser Asn Ser Ala Ala Ala Asp Ser His Leu Ser Ser Leu
    370                 375                 380

Ile Ser His Leu Leu His Leu Ala Ala His Val Glu Ser Phe Ser Ser
385                 390                 395                 400

His Pro Ile Ala Ala Ala Leu Arg Asp Ala Phe Pro Glu Ala Ala His
                405                 410                 415

Asp Asp Cys Glu Val Ser Asp Ile Lys Glu Ile Ala Gly His Gly Ile
            420                 425                 430

Gln Ala Arg Val Gly Asn Asp Ile Val Cys Val Gly Asn Thr Lys Met
        435                 440                 445

Met Asp Ser Leu Gly Val Ala Trp His Asp Cys His His Pro Gly Thr
    450                 455                 460

Ile Ile His Val Ala Ile Asn Gly Glu Tyr Ala Gly His Ile Ile Ile
465                 470                 475                 480

Asn Asp Met Ile Lys Asp Asp Ser Ala Glu Ala Ile Arg Gln Leu Lys
                485                 490                 495

Glu Leu Gly Val Glu Arg Thr Val Met Leu Thr Gly Asp Arg Lys Glu
            500                 505                 510

Val Ala Asp His Val Ala Lys Met Leu Asp Ile Thr Glu Tyr His Ala
        515                 520                 525

Glu Leu Leu Pro Thr Asp Lys Val Ser Phe Val Glu Gln Glu Leu Ser
    530                 535                 540

Ala His Ser Ser Ser Thr Ser His Pro Ser Tyr Val Ala Phe Val Gly
545                 550                 555                 560

Asp Gly Ile Asn Asp Ala Pro Val Leu Ala Arg Ala Asp Val Gly Ile
                565                 570                 575

Ala Met Gly Gly Leu Gly Ser Asp Ala Ala Ile Glu Ala Ala Asp Val
            580                 585                 590

Val Leu Met Asp Asp Gln Pro Ser Lys Ile Ala Gln Ala Ile Arg Ile
        595                 600                 605

Ala Arg Arg Thr Leu Ser Ile Ala Arg Gln Asn Val Trp Phe Ala Ile
    610                 615                 620

Gly Val Lys Ile Ala Val Leu Ile Leu Ala Thr Phe Gly Leu Ser Thr

625                 630                 635                 640

Met Trp Met Ala Val Phe Ala Asp Val Gly Val Thr Val Leu Ala Val
                645                 650                 655

Leu Asn Ala Met Arg Thr Met Tyr
            660

<210>  17
<211>  324
<212>  PRT
<213>  Unknown

<220>
<223>  Description of sequence: sequence derived from genomic DNA library
       from rumen ecosystem, particularly amino acid sequence of the
       polypeptide and the mature protein of clone pBKRR 7 mature protein
       (according to the protein homology analysis) ;(see Figure 20)

<400>  17

Val Lys Thr Val Val Phe Asp Lys Thr Gly Thr Leu Thr His Gly Gln
1               5                   10                  15

Phe Ala Val Glu Ala Val His Pro Asn Gln Gly Glu Ser Ser Cys Ser
            20                  25                  30

Asn Ser Ala Ala Ala Asp Ser His Leu Ser Ser Leu Ile Ser His Leu
        35                  40                  45

Leu His Leu Ala Ala His Val Glu Ser Phe Ser Ser His Pro Ile Ala
    50                  55                  60

Ala Ala Leu Arg Asp Ala Phe Pro Glu Ala Ala His Asp Asp Cys Glu
65                  70                  75                  80

Val Ser Asp Ile Lys Glu Ile Ala Gly His Gly Ile Gln Ala Arg Val
                85                  90                  95

Gly Asn Asp Ile Val Cys Val Gly Asn Thr Lys Met Met Asp Ser Leu
            100                 105                 110

Gly Val Ala Trp His Asp Cys His His Pro Gly Thr Ile Ile His Val
        115                 120                 125

Ala Ile Asn Gly Glu Tyr Ala Gly His Ile Ile Ile Asn Asp Met Ile
    130                 135                 140

Lys Asp Asp Ser Ala Glu Ala Ile Arg Gln Leu Lys Glu Leu Gly Val
145                 150                 155                 160

Glu Arg Thr Val Met Leu Thr Gly Asp Arg Lys Glu Val Ala Asp His
                165                 170                 175

Val Ala Lys Met Leu Asp Ile Thr Glu Tyr His Ala Glu Leu Leu Pro
            180                 185                 190

Thr Asp Lys Val Ser Phe Val Glu Gln Glu Leu Ser Ala His Ser Ser
        195                 200                 205

Ser Thr Ser His Pro Ser Tyr Val Ala Phe Val Gly Asp Gly Ile Asn
    210                 215                 220

Asp Ala Pro Val Leu Ala Arg Ala Asp Val Gly Ile Ala Met Gly Gly
225                 230                 235                 240

Leu Gly Ser Asp Ala Ala Ile Glu Ala Ala Asp Val Val Leu Met Asp
                245                 250                 255

Asp Gln Pro Ser Lys Ile Ala Gln Ala Ile Arg Ile Ala Arg Arg Thr
            260                 265                 270

Leu Ser Ile Ala Arg Gln Asn Val Trp Phe Ala Ile Gly Val Lys Ile
        275                 280                 285

Ala Val Leu Ile Leu Ala Thr Phe Gly Leu Ser Thr Met Trp Met Ala
    290                 295                 300

Val Phe Ala Asp Val Gly Val Thr Val Leu Ala Val Leu Asn Ala Met
305                 310                 315                 320

Arg Thr Met Tyr

<210>  18
<211>  353
<212>  PRT
<213>  Unknown

<220>
<223>  Description of sequence: sequence derived from genomic DNA library
       from rumen ecosystem, particularly amino acid sequence of clone
       pBKRR 9
; RR09;(see Figure 21)

<400>  18

Met Lys Leu Val Glu Phe Asn Ala Glu Glu Asn Tyr Ile Glu Asp Phe
1               5                   10                  15

Leu Ser Leu Pro Lys Lys Leu Tyr Thr Lys Leu Asp Asn Met Glu Asp
            20                  25                  30

Ser Asn Thr Met Lys Asp Ile Leu Thr Asn Asn His Pro Leu Ser Asn
        35                  40                  45

Asp Phe Lys Leu Asn Lys Tyr Leu Val Tyr Lys Asp Asp Glu Val Val
    50                  55                  60

Ala Arg Phe Ile Ile Thr Glu Tyr Asn Asp Asp Asn Lys Val Cys Tyr
65                  70                  75                  80

Ile Gly Phe Phe Glu Cys Ile Asn Asp Lys Lys Val Ala Lys Phe Leu
                85                  90                  95

Phe Asp Glu Ala His Lys Ile Ala Lys Glu Lys Lys Tyr Lys Lys Ile
            100                 105                 110

Ile Gly Pro Val Asp Ala Ser Phe Trp Ile Lys Tyr Arg Leu Lys Ile
        115                 120                 125

Asn Lys Phe Glu Arg Pro Tyr Thr Gly Glu Pro Tyr Asn Lys Asp Tyr
    130                 135                 140

Tyr Leu Lys Leu Phe Thr Asp Asn Lys Tyr Lys Ile Cys Asp His Tyr
145                 150                 155                 160

Thr Ser Gln Ser Tyr Asp Ile Val Asp Asp Ser Tyr Phe Asn Asp Lys
                165                 170                 175

Phe Thr Glu His Tyr Asn Glu Phe Lys Lys Leu Gly Tyr Glu Ile Ile
            180                 185                 190

Lys Pro Lys Pro Glu Asp Phe Asp Lys Cys Met Ser Glu Val Tyr Asp
        195                 200                 205

Leu Ile Thr Lys Leu Tyr Ser Asp Phe Pro Ile Phe Lys Asn Leu Lys
    210                 215                 220

Lys Glu Ser Phe Leu Glu Ile Tyr Lys Ser Tyr Lys Lys Ile Ile Asn
225                 230                 235                 240

Met Asn Met Val Arg Met Ala Tyr Phe Lys Gly Gln Ala Val Gly Phe
                245                 250                 255

Tyr Ile Ser Val Pro Asn Tyr Asn Asn Ile Val Tyr His Ile Asn Pro
            260                 265                 270

Ile Asn Ile Leu Lys Ile Leu His Gln Arg Lys His Pro Lys Gly Tyr
        275                 280                 285

Val Met Leu Tyr Met Gly Val Asp Ser Asn His Arg Gly Leu Gly Lys
    290                 295                 300

Ala Leu Val Tyr Ser Ile Val Glu Glu Leu Lys Lys Asn Asn Leu Pro
305                 310                 315                 320

Ser Ile Gly Ala Leu Ala His Asp Gly Lys Ile Ser Gln Asn Tyr Ala
                325                 330                 335

Lys Glu Lys Ile Asn Ser Arg Tyr Glu Tyr Val Leu Leu Glu Arg Ser
            340                 345                 350

Ile

<210>  19
<211>  536
<212>  PRT
<213>  Unknown

<220>
<223>  Description of sequence: sequence derived from genomic DNA library
       from rumen ecosystem, particularly amino acid sequence of clones
       pBKRR 11, 10 and 12
; RR10=RR11=RR12;(see Figure 22)

<400>  19

Met Arg Ile Leu Lys Leu Phe Phe Ile Ala Leu Ile Thr Ala Ala Pro
1               5                   10                  15

Ile Phe Ser Tyr Ala Gln Thr Val Asp Asp Met Lys Ser Val Phe Val
            20                  25                  30

Asp Ala His Thr Trp Thr Ala Ser Ile Lys Met Gly Trp Asn Leu Gly
        35                  40                  45

Asn Ala Leu Glu Cys Gln Thr Gly Trp Asn Asp Lys Ala Phe Cys Trp
    50                  55                  60

Asn Pro Ala Asn Asn Phe Asn Ala Glu Thr Ser Trp Gly Asn Pro Lys
65                  70                  75                  80

Thr Thr Lys Glu Met Leu Gln Ala Val Arg Glu Ala Gly Phe Asp Ala
                85                  90                  95

Val Arg Ile Pro Val Asn Trp Gly Ala His Ile Ser Asp Glu Ser Thr
            100                 105                 110

Cys Thr Ile Asp Ala Ala Trp Met Asn Arg Val Gln Glu Val Val Asp
        115                 120                 125

Trp Ala Leu Gln Leu Gly Phe Lys Val Leu Leu Asn Thr His His Glu
    130                 135                 140

Tyr Trp Leu Glu Trp His Pro Thr Tyr Asp Arg Glu Gln Ala Asn Asn
145                 150                 155                 160

Asn Lys Leu Ala Lys Ile Trp Met Gln Val Ala Asn Arg Phe Lys Asp
                165                 170                 175

Tyr Gly Pro Asp Leu Ala Phe Ala Gly Ile Asn Glu Val His Leu Asp
            180                 185                 190

Gly Lys Trp Asn Val Pro Thr Glu Glu Asn Thr Ala Val Thr Asn Ser
        195                 200                 205

Tyr Asn Gln Thr Phe Val Asn Thr Val Arg Ala Thr Gly Gly Asn Asn
    210                 215                 220

Leu Tyr Arg Asn Leu Val Val Gln Cys Tyr Ser Cys Asn Pro Asp Tyr
225                 230                 235                 240

Gly Leu Thr Gly Phe Val Val Pro Ala Asp Lys Val Glu Asn Arg Leu
                245                 250                 255

Ser Ile Glu Tyr His Tyr Tyr Arg Pro Trp Asp Tyr Ala Gly Asp Ala
            260                 265                 270

Pro Lys Tyr Tyr Tyr Trp Gly Glu Ala Tyr Lys Gln Tyr Gly Glu Val
        275                 280                 285

Ser Thr Ala Asp Asn Glu Ala Lys Val Asn Ala Asp Phe Ala Arg Tyr
    290                 295                 300

Lys Ala Ala Trp Tyr Asp Lys Gly Leu Gly Val Ile Met Gly Glu Trp
305                 310                 315                 320

Gly Ala Ser Gln His Tyr Gln Val Glu Ser Lys Ala Lys Gln Leu Glu
                325                 330                 335

Asn Leu Tyr Tyr His Phe Lys Thr Val Arg Glu Ala Ala Gln Lys Asn
            340                 345                 350

Asn Ile Ala Thr Phe Val Trp Asp Asn Asn Ala Cys Gly Asn Gly Gly
        355                 360                 365

Asp Lys Phe Gly Ile Phe Asp Arg Asn Asp Asn Met Ser Val Ala Phe
    370                 375                 380

Pro Glu Ala Leu Asn Ala Ile Gln Glu Ala Ser Gly Gln Thr Val Val
385                 390                 395                 400

Asp Tyr Ser Arg Gln Ala Leu Gln Pro Ser Val Val Tyr Gln Gly Asp
                405                 410                 415

Glu Leu Met Asn Trp Gly Glu Gly Lys Gln Leu Ile Ile Ala Gly Ser
            420                 425                 430

Lys Phe Ala Tyr Phe Thr Ala Glu Ser Lys Leu Met Val Thr Leu Asp
        435                 440                 445

Ala Glu Pro Gly Ala Asp Tyr Asp Met Leu Gln Phe Ala Tyr Gly Asp
    450                 455                 460

Trp Lys Ser Lys Pro Leu Met Ile Ile Ser Gly Arg Ser Tyr Lys Gly
465                 470                 475                 480

Gln Val Glu Pro Ser Lys Ile Asn Gly Ser Arg Asn Thr Tyr Thr Leu
                485                 490                 495

Phe Ile Gly Phe Lys Glu Ser Ser Leu Asn Gln Leu Lys Asp Lys Gly
            500                 505                 510

Ile Thr Leu Gln Gly His Gly Leu Arg Leu Arg Asn Val Val Val Met
        515                 520                 525

Pro Glu Gly Leu Val Leu Gly Leu
    530                 535

<210>  20
<211>  320
<212>  PRT
<213>  Unknown

<220>
<223>  Description of sequence: sequence derived from genomic DNA library
       from rumen ecosystem, particularly amino acid sequence of clone
       pBKRR 13
; RR 13;(see Figure 23)

<400>  20

Met Thr Met Lys Lys Leu Leu Thr Ile Ile Met Leu Ser Leu Leu Ala

1               5                   10                  15

Cys His Val His Ala Asn Asp Pro Val Lys Gln Tyr Gly Lys Leu Gln
            20                  25                  30

Val Lys Gly Ala Gln Leu Cys Asp Gln Lys Gly Asn Pro Val Ile Leu
        35                  40                  45

Arg Gly Val Ser Leu Gly Trp His Asn Leu Trp Pro Arg Phe Tyr Asn
    50                  55                  60

Lys Gly Ala Val Glu Thr Leu Lys Asn Asp Trp His Cys Ser Val Ile
65                  70                  75                  80

Arg Ala Ala Ile Gly Gln His Ile Glu Asp Asn Phe Gln Glu Asn Pro
                85                  90                  95

Glu Phe Ala Met Gln Cys Leu Thr Pro Val Val Glu Ala Ala Ile Lys
            100                 105                 110

Gln Asn Val Tyr Val Ile Ile Asp Trp His Ser His Lys Leu Met Thr
        115                 120                 125

Glu Glu Ala Lys Gln Phe Phe Gly Glu Met Ala Arg Lys Tyr Gly Lys
    130                 135                 140

Tyr Pro His Ile Ile Tyr Glu Ile Tyr Asn Glu Pro Ile Ala Asp Thr
145                 150                 155                 160

Trp Thr Asp Leu Lys Lys Tyr Ala Gln Glu Val Ile Gly Glu Ile Arg
                165                 170                 175

Lys Tyr Asp Lys Asp Asn Val Val Leu Val Gly Cys Pro His Trp Asp
            180                 185                 190

Gln Asp Ile His Leu Val Ala Glu Ser Pro Leu Glu Gly Leu Ser Asn
        195                 200                 205

Val Met Tyr Thr Val His Phe Tyr Ala Ala Thr His Gly Glu Asp Leu
    210                 215                 220

Arg Gln Arg Thr Glu Glu Ala Ala Lys Arg Gly Ile Pro Ile Phe Ile
225                 230                 235                 240

Ser Glu Ser Gly Ala Thr Glu Ala Ser Gly Asp Gly Lys Ile Asp Glu
                245                 250                 255

Glu Ser Glu Glu Glu Trp Ile Lys Met Cys Glu Arg Leu Gly Ile Ser
            260                 265                 270

Trp Leu Cys Trp Ser Ile Ser Asp Lys Asn Glu Ser Ser Ser Met Leu
        275                 280                 285

Leu Pro Arg Ala Thr Ala Thr Gly Pro Trp Pro Asp Asp Val Ile Lys
    290                 295                 300

Lys Tyr Gly Lys Leu Val Lys Gly Leu Leu Glu Lys Tyr Asn Ala Arg
305                 310                 315                 320

<210>  21
<211>  483
<212>  PRT
<213>  Unknown

<220>
<223>  Description of sequence: sequence derived from genomic DNA library
       from rumen ecosystem, particularly amino acid sequence of clones
       pBKRR 14 and 20-1
; RR14=RR20-1;(see Figure 24)

<400>  21

Met Ile Asn Val Leu Gln His His Pro Glu Val Glu Leu Ser Val Asp
1               5                   10                  15

Lys Thr Ser Val Ser Phe Asn Arg Ser Gly Gly Glu Glu Thr Phe Thr
            20                  25                  30

Val Thr Ser Ser Thr Gln Pro His Val Ser Ala Asp Val Ser Trp Val
        35                  40                  45

Val Val Glu Thr Gly Lys Ile Asp Lys Asp His His Thr Glu Val Arg
    50                  55                  60

Val Leu Ala Gly Ala Asn Arg Lys Glu Ala Ser Ala Gly Thr Leu Thr
65                  70                  75                  80

Val Ser Cys Ser Asp Lys Lys Val Ser Val Ser Val Lys Gln Glu Ala
                85                  90                  95

Phe Val Ala Pro Ser Val Ala Ser Thr Thr Ala Val Thr Pro Gln Met
            100                 105                 110

Val Phe Asp Ala Met Gly Pro Gly Trp Asn Met Gly Asn His Met Asp
        115                 120                 125

Ala Ile Ser Asn Gly Val Ser Gly Glu Thr Val Trp Gly Asn Pro Lys

130                 135                 140

Cys Thr Gln Ala Thr Met Asp Gly Val Lys Ala Ala Gly Tyr Lys Ala
145                 150                 155                 160

Val Arg Ile Cys Thr Thr Trp Glu Gly His Ile Gly Ala Ala Pro Ala
                165                 170                 175

Tyr Ala Leu Glu Gln Lys Trp Leu Asp Arg Val Ala Glu Ile Val Gly
            180                 185                 190

Tyr Ala Glu Lys Ala Gly Leu Val Ala Ile Val Asn Thr His His Asp
        195                 200                 205

Glu Ser Tyr Trp Gln Asp Ile Ser Lys Cys Tyr Asn Asn Ala Ala Asn
    210                 215                 220

His Glu Lys Val Lys Asp Glu Val Phe Ser Val Trp Thr Gln Ile Ala
225                 230                 235                 240

Glu Lys Phe Lys Asp Lys Gly Glu Trp Leu Val Phe Glu Ser Phe Asn
                245                 250                 255

Glu Ile Gln Asp Gly Gly Trp Gly Trp Ser Asp Ala Phe Arg Lys Asn
            260                 265                 270

Pro Asp Ala Gln Tyr Lys Val Leu Asn Glu Trp Asn Gln Thr Phe Val
        275                 280                 285

Asp Ala Val Arg Ser Thr Gly Gly Gln Asn Ala Thr Arg Trp Leu Gly
    290                 295                 300

Ile Pro Gly Tyr Ala Cys Asn Pro Gly Phe Thr Ile Ala Gly Leu Val
305                 310                 315                 320

Leu Pro Lys Asp Tyr Thr Thr Ala Asn Arg Leu Met Val Ala Val His
                325                 330                 335

Asp Tyr Asp Pro Tyr Asp Tyr Thr Leu Lys Asp Pro Leu Ile Arg Gln
            340                 345                 350

Trp Gly His Thr Ala Asp Ala Asp Lys Arg Pro Ser Gly Asp Asn Glu
        355                 360                 365

Lys Ala Val Val Asp Val Phe Asn Asn Leu Lys Ala Ala Tyr Leu Asp
    370                 375                 380

Lys Gly Ile Pro Val Tyr Leu Gly Glu Met Gly Cys Ser Arg His Thr
385                 390                 395                 400

Ala Ala Asp Phe Pro Tyr Gln Lys Tyr Tyr Met Glu Tyr Phe Cys Lys
                405                 410                 415

Ala Ala Ala Asp Arg Leu Leu Pro Met Tyr Leu Trp Asp Asn Gly Ala
            420                 425                 430

Lys Gly Val Gly Ser Glu Arg His Ala Tyr Ile Asp His Gly Thr Gly
        435                 440                 445

Gln Phe Val Asp Glu Asp Ala Arg Thr Leu Val Gly Leu Met Val Lys
    450                 455                 460

Ala Val Thr Thr Lys Asp Ala Ser Tyr Thr Leu Glu Ser Val Tyr Asn
465                 470                 475                 480

Ser Ala Pro

## Description

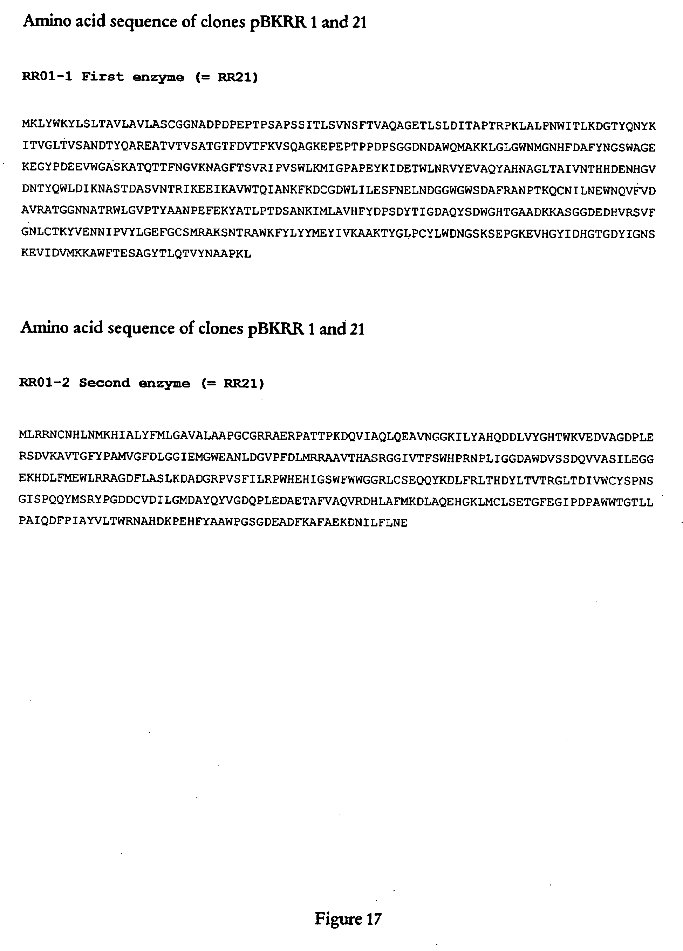

The invention relates to new cellulases from rumen, in particular to polypeptides comprising or consisting of an amino acid sequence according to Figure 17 to 24 of the invention or parts thereof or a functional fragment or functional derivative thereof.

Cellulases or cellulolytic enzymes are enzymes which are involved in hydrolysis of cellulose, especially in hydrolysis of the 3-D-glucosidic linkages in cellulose. Cellulose is an unbranched polymer of 1,4-linked anhydrous glucose units of variable length, and is one of the most abundant natural polymers with an estimated annual production of 4x 10⁹t. Cellulose offers an energy and carbon source for some organism, particularly microorganism. The cellulose polymer itself cannot pass the membrane of microorganisms and has to be hydrolyzed prior to utilization. Therefore, cellulases are synthesized by a large number of microorganisms which include fungi, actinomycetes, myxobacteria and true bacteria but also by plants.

In the hydrolysis of native cellulose, it is known that there are three major types of cellulase enzymes involved which form a cellulolytic system and hydrolyze insoluble cellulose, cellulose derivatives (such as carboxymethyl cellulose and hydroxyethylcellulose), lichenin, beta-1,4 bonds in mixed beta-1,3 glucans such as cereal beta-D-glucans or xyloglucans and other plant material containing cellulosic parts in a very efficient and synergistic way. These three types of cellulose enzymes are:
(a) endo-beta-1,4-glucanases (endo-1,4-beta-D-glucan-4-glucanohydrolase, EC 3.2.1.4) which randomly hydrolyze the 1,4-glycosidyl linkages within the water-insoluble cellulose chains,
(b) exoglucanases or cellobiohydrolases (1,4-beta-D-glucan-cellobiohydrolase, EC 3.2.1.91) which hydrolyze the 1,4-glycosidyl linkages at either the reducing or nonreducing ends of cellulose chains to form cellobiose (glucose dimer) and
(c) beta-glucosidases or cellobioses (EC 3.2.1.21) which convert the water soluble cellobiose into two glucose residues.

Especially type (a) the endo-beta-1,4-glucanases constitute an interesting group of hydrolases for industrial uses and thus a wide variety of specificities have been identified (T-M Enveri, Microbial Cellulases in W. M. Fogarty, Microbial Enzymes and Biotechnology, Applied Sciences Publishers, p. 183-224 (1983); Methods in Enzymology, (1988) Vol. 160, p. 200-391 (edited by Wood, W. A. and Kellogg, S. T.); Béguin, P., Molecular Biology of Cellulose Degradation, Annu. Rev. Microbiol. (1990), Vol. 44, pp. 219-248; Béguin, P. and Aubert, J-P., The biological degradation of cellulose, FEMS, Microbiology Reviews 13 (1994) p. 25-58; Henrissat, B., Cellulases and their interaction with cellulose, Cellulose (1994), Vol. 1, pp. 169-196). Industrially well-performing endo-beta-1,4-glucanases are described in e.g. WO 91/17243, WO 91/17244 and WO 91/10732 and specific cellulase variants are described in WO 94/07998.

Cellulases or cellulolytic enzymes are mainly used for industrial purposes. Such industrial uses include, for example, the use in consumer products and food industries, e.g. in extracting and clarifying juice from fruits or vegetables. Another important industrial use of cellulases or cellulolytic enzymes is their use for treatment of paper pulp, e.g. for improving the drainage or for deinking of recycled paper. Even further cellulase applications include biodiesel production, insofar cellulases are needed to convert cellulosic biomass to fermentable sugars which are a valuable source for the production of chemicals and fuels, syrup production and agrobiotechnological processes, such as bioprocessing of crops and crop residues, fibre processing and production of feed supplements to improve feed efficiency.

However, among many applications which have been developed for the use of cellulolytic enzymes a very important industrial use of cellulases or cellulolytic enzymes is the use for treatment of cellulosic textile or fabrics, e.g. as indegrients in detergent compositions or fabric softener compositions for biopolishing of new fabrics (garment finish) or for obtaining a stone-washed look of cellulose containing fabrics, especially denim. Therefore, cellulases are known to be useful in detergent compositions for removing dirt, i.e., cleaning. For example, GB Application Nos. 2,075,028, 2,095,275 and 2,094,826 illustrate improved cleaning performance when detergents incorporate cellulases. Additionally, GB Application No. 1,358,599 teaches the use of cellulases in detergents to reduce the harshness of cotton containing fabrics. Another useful feature of cellulases in the treatment of textiles is their ability to recondition used fabrics by making their colors more vibrant. For example, repeated washing of cotton containing fabrics results in a greyish cast to the fabrics which is believed to be due to disrupted and disordered fibrils caused by mechanical action. This greyish cast is particularly noticeable on colored fabrics. As a consequence, the ability of cellulase to remove the disordered top layer of the fiber and thus improve the overall appearance of the fabrics has been of value.

Since detergents containing cellulases operate generally under alkaline conditions, there is a strong demand for cellulases which have excellent activity at pH 7-10. Well characterized fungal cellulases, such as those from *Humicola insolens* and *Trichoderma reesei,* perform adequately at neutral to low alkaline pH. Further, a number of bacterial enzymes that show cellulase activity at high alkaline pH have been isolated from *Bacillus* and other prokaryotes, see e.g., WO 96/34092 and WO 96/34108. Furthermore, Wilson et aL, Critical Reviews in Biotechnology, Vol. 12, pp. 45-63 (1992), studied cellulases produced by *Thermomonospora fusca, Themonomospora curvata* and *Microbispora bispora* and discovered that many of these cellulases show broad pH profiles and good temperature stability. Similarly, Nakai et al., Agric. Biol. Chem., Vol. 51, pp. 3061- 3065 (1987) and Nakai et al., Gene, VoL 65, pp. 229-238 (1988) exemplify the alkalitolerant cellulase casA from *Streptomyces* strain KSM-9 which also possesses an alkaline pH optimum and good temperature stability.

The enzymatic properties of some of the investigated cellulases in the art may fulfill the essential requirements upon which various industrial processes are based. However, apart from a high alkaline pH and a good temperature stability other properties are desirable for an effective application. Other properties are e.g., optimal phenotype of expression, temperature stability and pH optimum, high stability and high specific activity towards cellulose substrates, e.g. detergents, high enzyme rate and low addition of cations, because removal of these compounds is an expensive step of the overall process costs.

Despite vast information available in the art about numerous cellulases having desirable properties for certain applications, cellulases, cellulase compositions or cellulolytic enzymes which simultaneously exhibit some or even most of the aforementioned properties are not known.

Therefore, it is an object of the present invention to provide cellulases with improved properties, preferably a combination of improved properties, which are useful for industrial applications.

As a solution to the aforementioned object new cellulases which possess improved properties and which are advantageous for known applications of cellulose have been discovered. Therefore, the present invention relates in its first embodiment to a polypeptide comprising one of the amino acid sequences of amino acids No. 175 to No. 210 of the sequences shown in Figures 17 to 24 or a functional fragment, or functional derivative thereof.

Preferably, the polypeptide of the invention comprises one of the amino acid sequences of amino acids No. 175 to 210, preferably No. 170 to No. 220, more preferably No. 150 to 240, most preferably No. 120 to 280 of the sequences shown in Figures 17 to 24. More preferably, the polypeptide of the invention comprises one of the amino acid sequences shown in Figures 17 to 24.

The invention is based on the discovery that rumen ecosystems represent a unique microbial ecosystem with a high potential of microbial and manifold enzymatic diversity including, e.g., cellulases, hemicelluloses, xylases, glucosidases, endoglucanases etc.. Therefore, rumen ecosystems containing a wide variety of microorganisms form a good starting material for screening new cellulases to obtain new cellulolytic activies. Consequently, according to a preferred embodiment of the invention, the polypeptide is derived from rumen, particularly from rumen ecosystem, preferably from cow rumen, more preferably from New Zealand dairy cow.

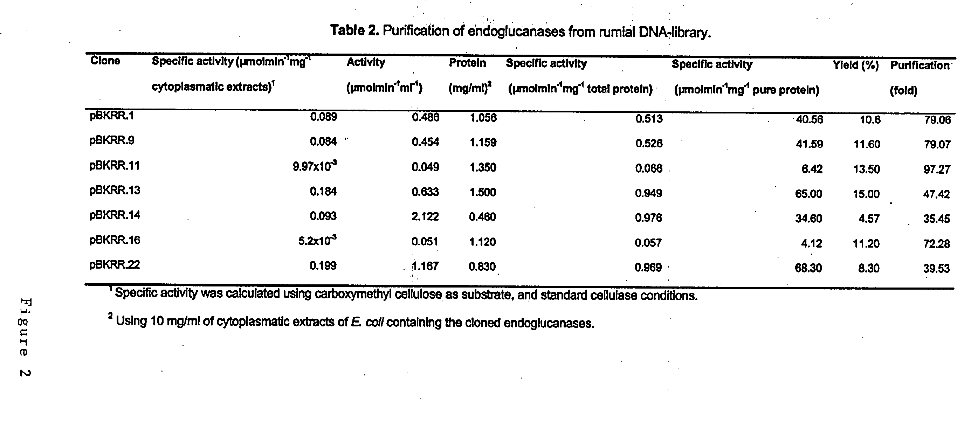

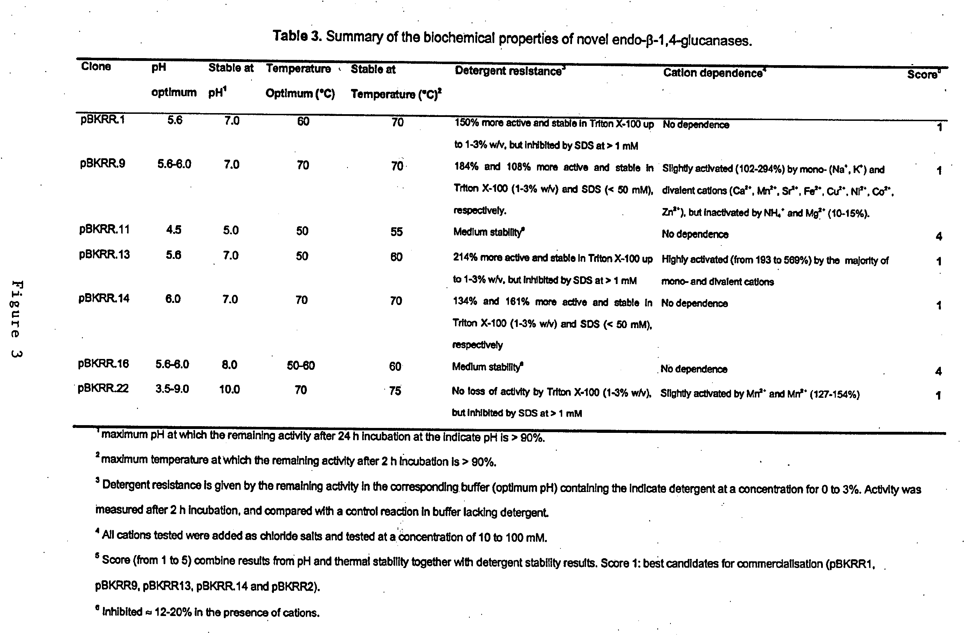

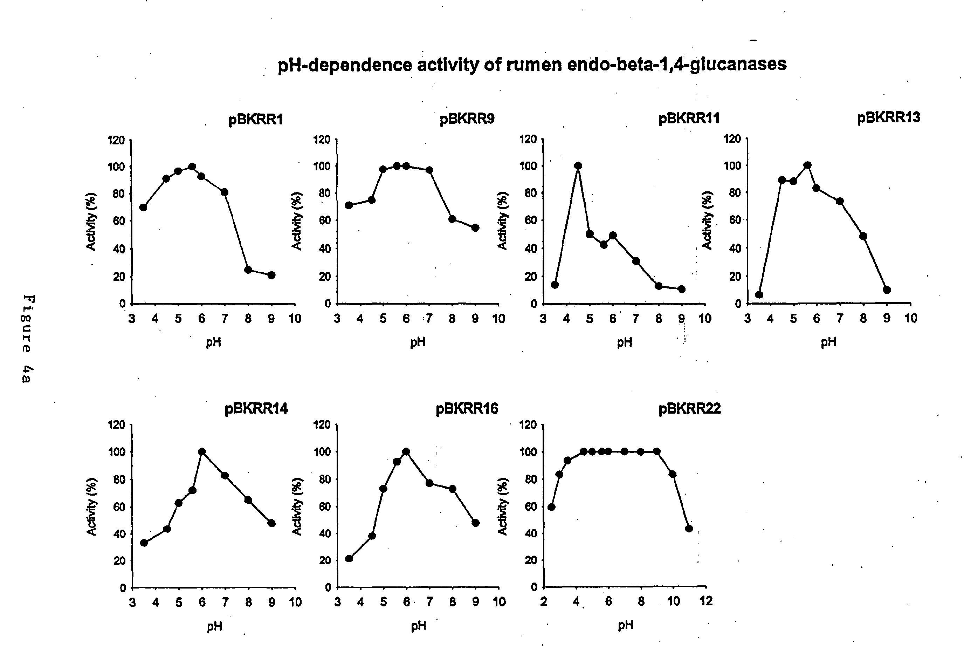

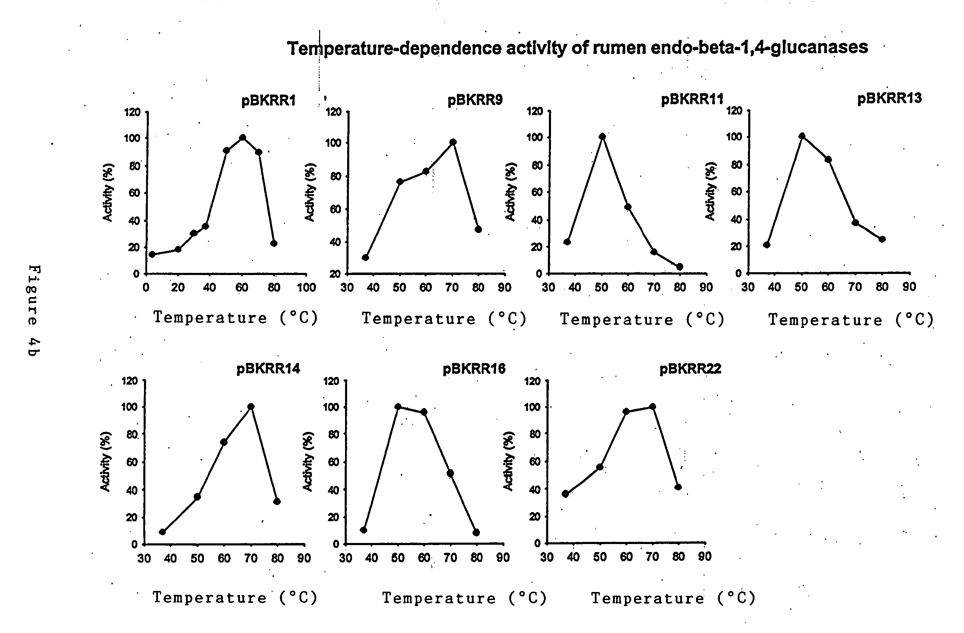

According to the invention it was possible to identify a group of endo-beta-1,4-glucanases from an expression library created previously from extracted rumial ecosystem-DNA. Especially, five novel high performance endo-beta-1,4-glucanases which hydrolyze i.a. carboxymethyl cellulose (CMC) were defined. Most of the polypeptides of the invention show an endoglucanase activity which is considerably higher than of endoglucanases known in the art (see i.a. Figure 2, 3, 5, 6), are stable over a broad pH ranging from pH 3.5 to pH 10.0 and at a temperature of up to 70°C and are not influenced by mono- and divalent cations (see Figure 3).

Consequently, in preferred embodiments of the invention functional active polypeptides show activity at pH optimum, preferably at pH ranging from 3.5 to 10.0, more preferably from 3.5 to 5.5 or from 5.5 to 7.0 or from 6.5 to 9.0, most preferably from 7.0 to 10.0, at temperature optimum, preferably at a temperature from 40°C to 70°C, more preferably from 40°C to 60°C, most preferably from 50°C to 70°C and/or at low addition of cations , preferably without any addition of cations. Furthermore, it is preferred that the polypeptides show highly specific activities and/or high stability towards its substrate. Some or all of these features may be realized by functional active polypeptides of the invention. In a particularly preferred embodiment of the invention, the polypeptide shows a combination of at least two, preferably three, more preferably four, most preferably all five of the aforementioned features.

Activity, i.e. hydrolyzing activity of the polypeptide of the invention, at a "pH optimum" means that the polypeptide shows activity and is stable towards its substrate at a pH which is optimal for the individual application. For use at acidophil conditions it is desired to obtain stable and specific activity from about pH 3.5 to 4.0, whereas use at alkaline conditions (e.g., in detergent compositions) needs a pH optimum from 9.0 to 10.0.

Correspondingly, activity at a "temperature optimum" depends on the specific use of the functional active polypeptides of the invention and means that the polypeptides show activity and are stable towards their environment conditions at a temperature which is optimal for the respective application. Usually, a temperature stability is required from 40°C up to 70°C for the functional active polypeptides of the invention. For most industrial applications a temperature stability of the functional active polypeptide according to the invention at 70°C is preferred.

Most enzymes, particulary hydrolyzing or cellulolytic enzymes, require cations for their activity. Usually, mono- or divalent cations as NH₄⁺, K⁺, Na⁺ or Ca²⁺ Mn²⁺, Mg²⁺, Sr²⁺, Fe²⁺, Cu²⁺, Ni²⁺, Co²⁺, Zn²⁺ have to be added to obtain satisfying enzymatic activity. Thus, "activity at low addition of cations ", preferably activity without addition of cations, means that a polypeptide of the invention shows activity and is stable towards its environment conditions at a low cation concentration, preferably without addition of any cations at all.

"High specific activity" of the polypeptide of the invention means that its activity is essentially directed only towards its substrates.

"Functional", e.g. functional fragment or functional derivative according to the invention means that the polypeptides exhibits cellulolytic activity towards their substrates, particularly any hydrolytic effect on cellulase substrates. Especially, it relates to the hydrolysis of the 3-D-glucosidic linkages of cellulase substrates, e.g. cellulose, carboxymethyl cellulose, cellulose polymers etc., to shorter cello-oligosaccharide oligomers, cellobiose and/or glucose. Several methods for measuring cellulolytic activity are known by a person skilled in the art (e.g. enzyme assays using marked substrates, substrate analysis by chromatographic methods (as HPLC or TLC) for separating enzyme and substrate and spectrophotometric assays for measuring hydrolytic activity) (see e.g., Sambrook J, Maniatis T (1989) Molecular Cloning: A laboratory manual, Cold Spring Harbor Laboratory Press, Cold Spring Habor, NY). Among various alternatives, an appropriate method is, for example, the cellulase activity assay, as described below (see Example 5). This assay system is based on an activity selection technique with Ostazin-brilliant red-hydroxyethyl cellulose as assay substrate.

The term "fragment of a polypeptide" according to the invention is intended to encompass a portion of a amino sequence disclosed herein of at least about 60 contiguous amino acids, preferably of at least about 80 contiguous amino acids, more preferably of at least about 100 contiguous amino acids or longer in length. Functional fragments which encode polypeptides that retain their activity are particularly useful.

A "derivative of a polypeptide" according to the invention is intended to indicate a polypeptide which is derived from the native polypeptide by substitution of one or more amino acids at one or two or more of different sites of the native amino acid sequence, deletion of one or more amino acids at either or both ends of the native amino acid sequence or at one or more sites of the amino acid sequence, or insertion of one or more amino acids at one or more sites of the native amino acid sequence retaining its characteristic activity, particularly cellulolytic activity. Such a polypeptide can possess altered properties which may be advantageous over the properties of the native sequence for certain applications (e.g. increased pH optimum, increased temperature stability etc.).

A derivative of a polypeptide according to the invention means a polypeptide which has substantial identity with the amino acid sequences disclosed herein. Particularly preferred are nucleic acid sequences which have at least 60% sequence identity, preferably at least 75% sequence identity, even more preferably at least 80%, yet more preferably 90% sequence identity and most preferably at least 95% sequence identity thereto. Appropriate methods for isolation of a functional derivative of a polypeptide as well as for determination of percent identity of two amino acid sequences is described below.

The production of such polypeptide fragments or derivatives (as described below) is well known and can be carried out following standard methods which are well known by a person skilled in the art (see e.g., Sambrook J, Maniatis T (1989) *supra*). In general, the preparation of such functional fragments or derivatives of a polypeptide can be achieved by modifying a DNA sequence which encode the native polypeptide, transformation of that DNA sequence into a suitable host and expression of the modified DNA sequence to form the functional derivative of the polypeptide with the provision that the modification of the DNA does not disturb the characteristic activity, particularly cellulolytic activity.

The isolation of these polypeptide fragments or derivatives (as described below) can be carried out using standard methods as separating from cell or culture medium by centrifugation, filtration or chromatography and precipitation procedures (see, e.g., Sambrook J, Maniatis T (1989) *supra*)*.*

The polypeptide of the invention can also be fused to at least one second moiety. Preferably, the second or further moiety/moieties does not occur in the cellulase as found in nature. The at least second moiety can be an amino acid, oligopeptide or polypeptide and can be linked to the polypeptide of the invention at a suitable position, for example, the N-terminus, the C-terminus or internally. Linker sequences can be used to fuse the polypeptide of the invention with at least one other moiety/moieties. According to one embodiment of the invention, the linker sequences preferably form a flexible sequence of 5 to 50 residues, more preferably 5 to 15 residues. In a preferred embodiment the linker sequence contains at least 20%, more preferably at least 40% and even more preferably at least 50% Gly residues. Appropriate linker sequences can be easily selected and prepared by a person skilled in the art. Additional moieties may be linked to the inventive sequence, if desired. If the polypeptide is produced as a fusion protein, the fusion partner (e.g, HA, HSV-Tag, His6) can be used to facilitate purification and/or isolation. If desired, the fusion partner can then be removed from polypeptide of the invention (e.g., by proteolytic cleavage or other methods known in the art) at the end of the production process.

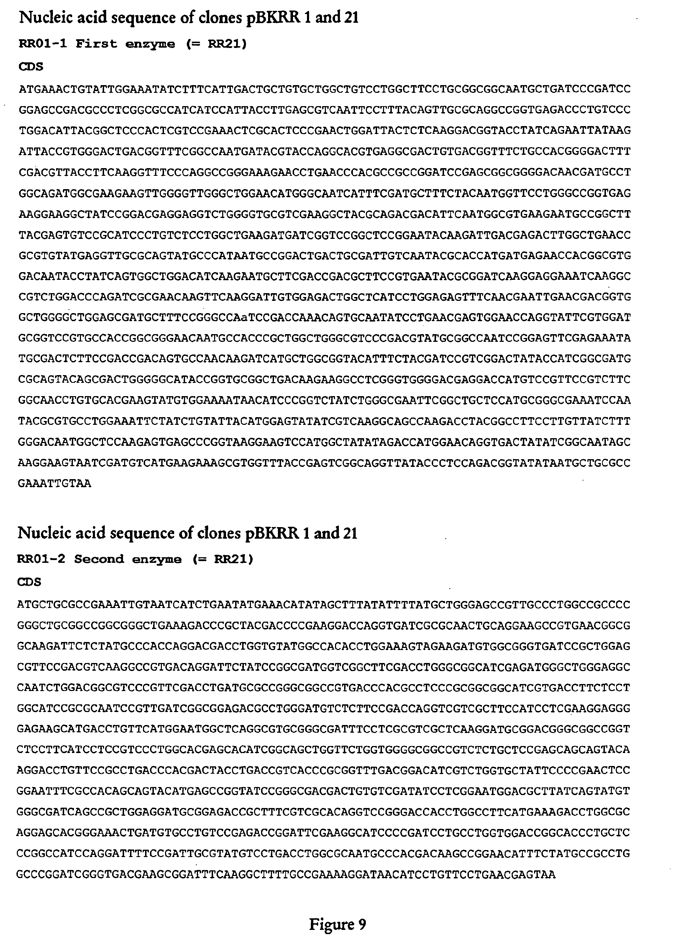

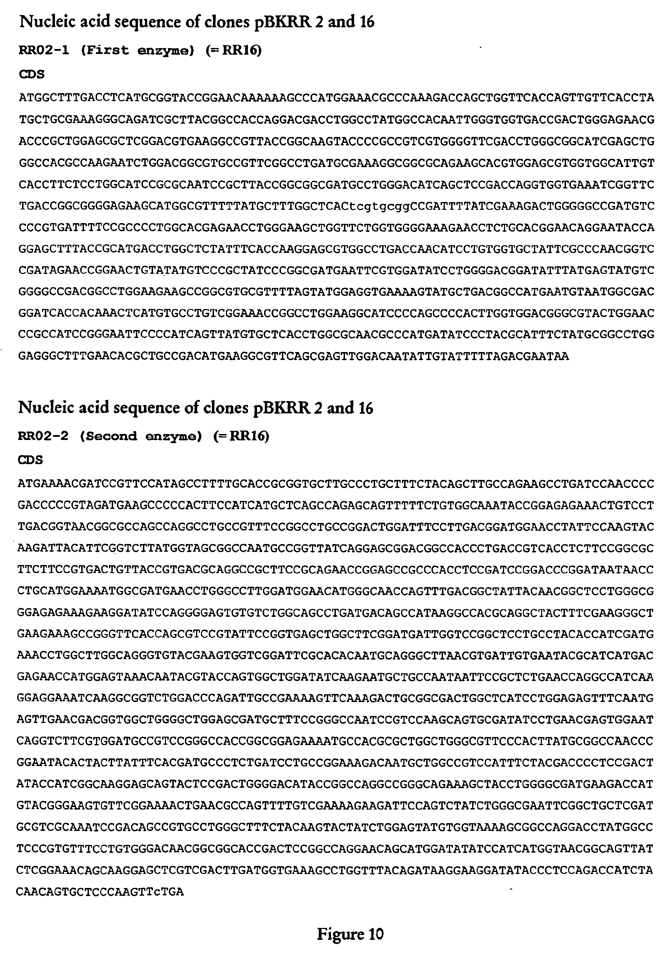

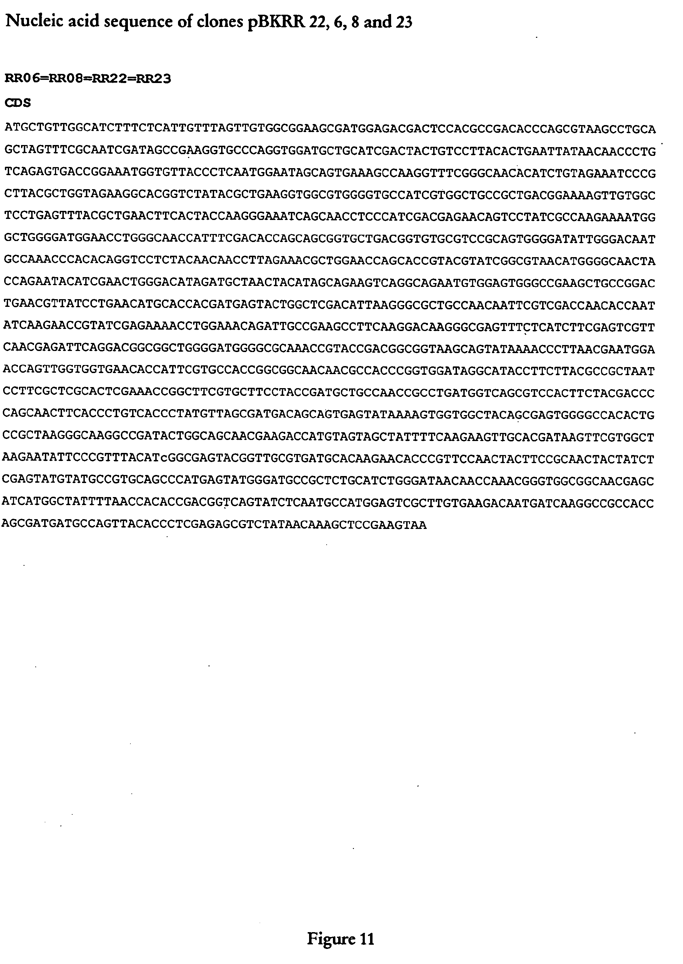

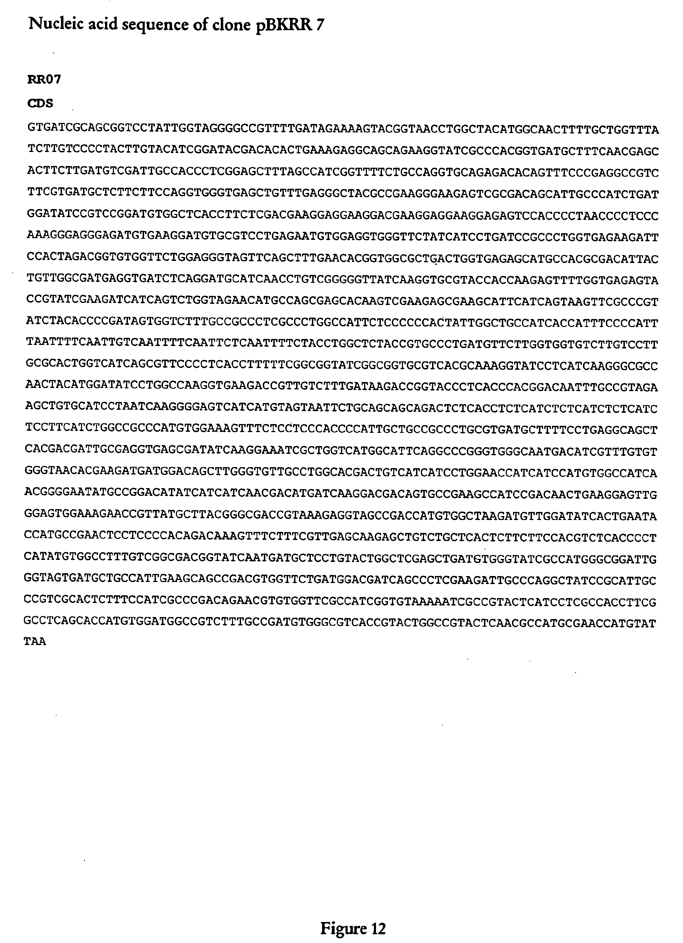

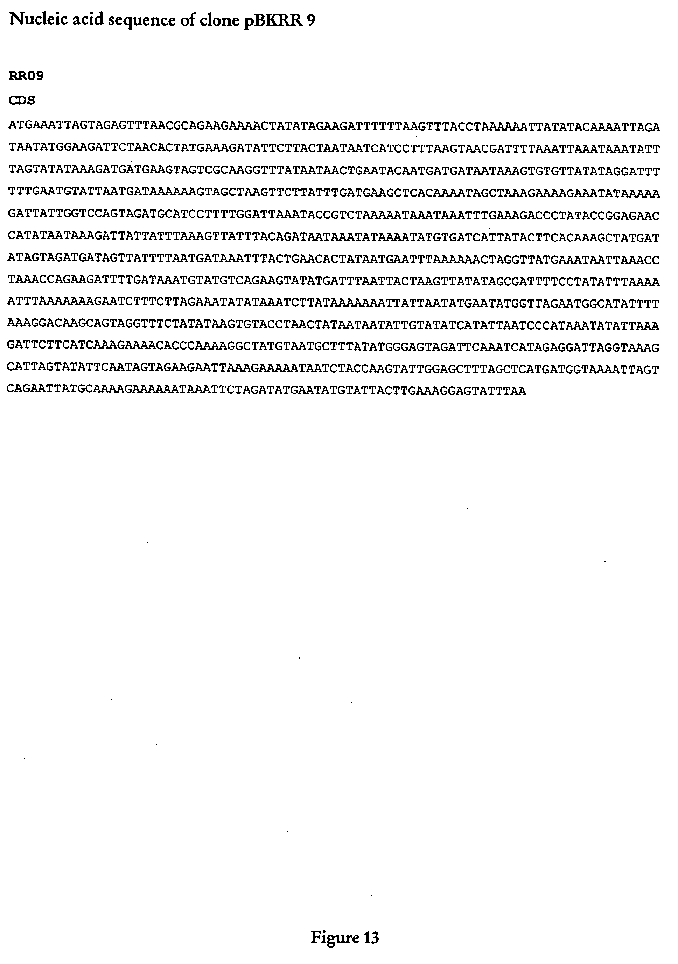

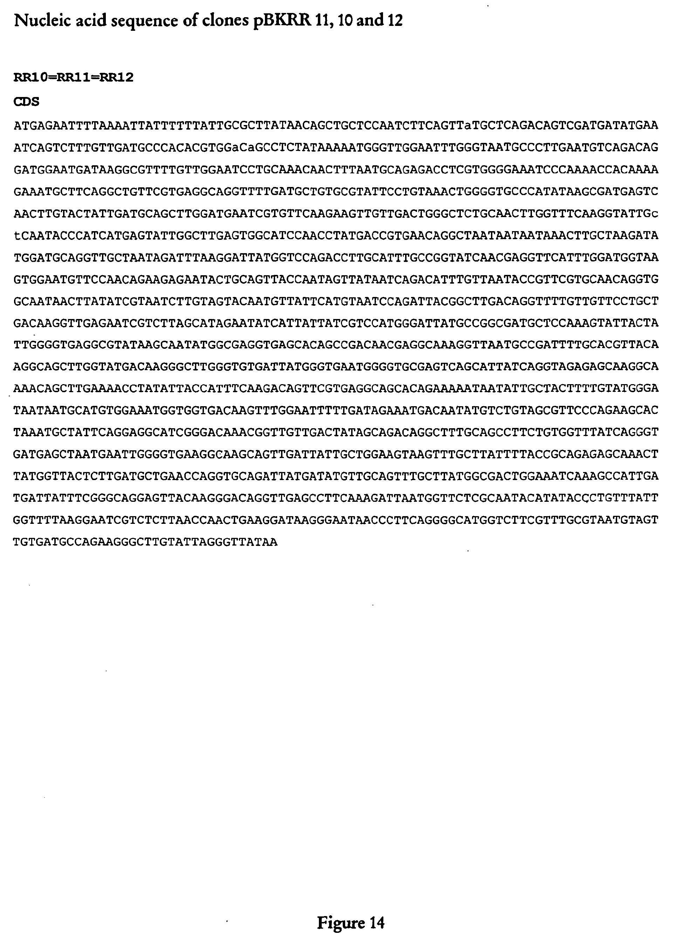

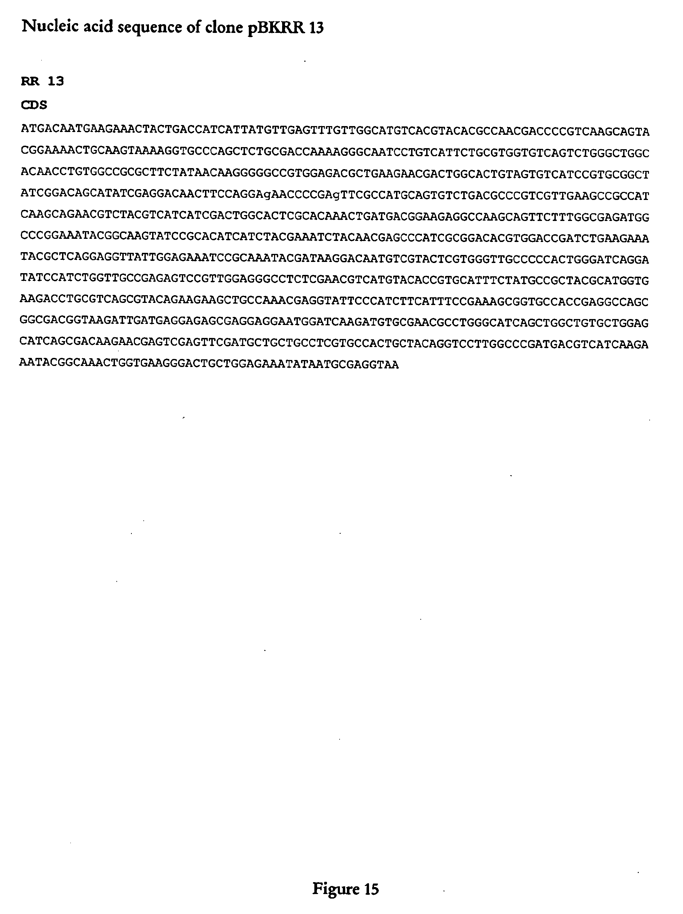

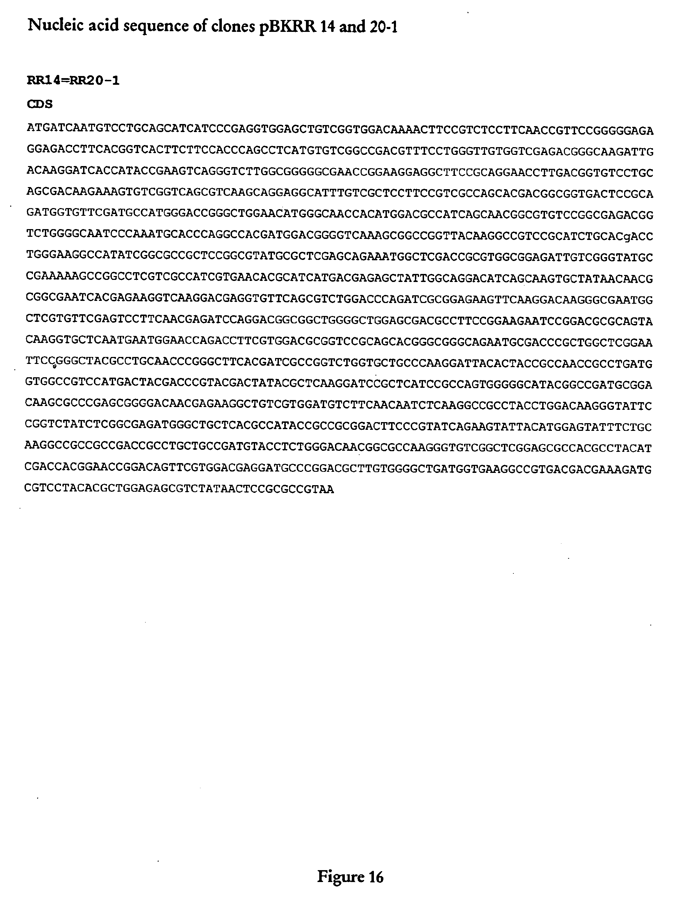

According to another embodiment of the invention a nucleic acid encoding a polypeptide of the invention (or a functional fragment or functional derivative thereof) or a functional fragment or functional derivative of said nucleic acid is provided. Preferably, the nucleic acid comprises or consists of one of the nucleic acid sequences of Figures 9 to 16.

The nucleic acids of the invention can be DNA or RNA, for example, mRNA. The nucleic acid molecules can be double-stranded or single-stranded; single stranded RNA or DNA can be either the coding (sense) strand or the non-coding (antisense) strand. If desired, the nucleotide sequence of the isolated nucleic acid can include additional non-coding sequences such as non-coding 3'- and 5'- sequences (including regulatory sequences, for example). All nucleic acid sequences, unless otherwise designated, are written in the direction from the 5' end to the 3' end.

Furthermore, the nucleic acids of the invention can be fused to a nucleic acid comprising, for example, a marker sequence or a nucleotide sequence which encodes a polypeptide to assist, e.g., in isolation or purification of the polypeptide. Representative sequences include, but are not limited to those which encode a glutathione-S-transferase (GST) fusion protein, a polyhistidine (e.g, His6), hemagglutinin, HSV-Tag, for example.

The term "nucleic acid" relates also to a fragment or derivative of said nucleic acid as described below.

The term "fragment of a nucleic acid" is intended to encompass a portion of a nucleotide sequence described herein which is from at least about 25 contiguous nucleotides to at least about 50 contiguous nucleotides, preferably at least about 60 contiguous nucleotides, more preferably at least about 120 contiguous nucleotides, most preferably at least about 180 contiguous nucleotides or longer in length. Especially, shorter fragments according to the invention are useful as probes and also as primer. Particularly preferred primers and probes selectively hybridize to the nucleic acid molecule encoding the polypeptides described herein. A primer is a nucleic acid fragment which functions as an initiating substrate for enzymatic or synthetic elongation. A probe is a nucleic acid sequence which hybridizes with a nucleic acid sequence of the invention, a fragment or a complementary nucleic acid sequence thereof. Fragments which encode polypeptides according to the invention that retain activity are particularly useful.

Hybridization can be used herein to analyze whether a given fragment or gene corresponds to the cellulase described herein and thus falls within the scope of the present invention. Hybridization describes a process in which a strand of nucleic acid joins with a complementary strand through base pairing. The conditions employed in the hybridization of two non-identical, but very similar, complementary nucleic acids varies with the degree of complementary of the two strands and the length of the strands. Such conditions and hybridisation techniques are well known by a person skilled in the art and can be carried out following standard hybridization assays (see e.g., SambrookJ, Maniatis T (1989) *supra*). Consequently, all nucleic acid sequences which hybridize to the nucleic acid or the functional fragments or functional derivatives thereof according to the invention are encompassed by the invention.

A "derivative of a nucleic acid" according to the invention is intended to indicate a nucleic acid which is derived from the native nucleic acid corresponding to the description above relating to a "functional derivative of a polypeptide", i.e. by addition, substitution, deletion or insertion of one or more nucleic acids retaining the characteristic activity, particularly cellulolytic activity of said nucleic acid. Such a nucleic acid can exhibit altered properties in some specific aspect (e.g. increased or decreased expression rate).

As skilled artisans will recognize that the amino acids of polypeptides of the invention can be encoded by a multitude of different nucleic acid triplets because most of the amino acids are encoded by more than one nucleic acid triplet due to the degeneracy of the amino acid code. Because these alternative nucleic acid sequences would encode the same amino acid sequences, the present invention further comprises these alternate nucleic acid sequences.

A derivative of a nucleic acid according to the invention means a nucleic acid or a fragment or a derivative thereof which has substantial identity with the nucleic acid sequences described herein. Particularly preferred are nucleic acid sequences which have at least about 30%, preferably at least about 40%, more preferably at least about 50%, even more preferably at least about 60%, yet more preferably at least about 80%, still more preferably at least about 90%, and even more preferably at least about 95% identity with nucleotide sequences described herein.

To determine the percent identity of two nucleotide sequences, the sequences can be aligned for optimal comparison purposes (e. g., gaps can be introduced in the sequence of a first nucleotide sequence). The nucleotides at corresponding nucleotide positions can then be compared. When a position in the first sequence is occupied by the same nucleotide as the corresponding position in the second sequence, then the molecules are identical at that position. The percent identity between the two sequences is a function of the number of identical positions shared by the sequences.

The determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program which can be used to identify sequences having the desired identity to nucleotide sequences of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The described method of determination of the percent identity of two can be also applied to amino acid sequences.

The production of such nucleic acid fragments or derivatives (as described below) is well known and can be carried out following standard methods which are well known by a person skilled in the art (see e.g., Sambrook J, Maniatis T (1989) *supra*). In general, the preparation of such functional fragments or derivatives of a nucleic acid can be achieved by modifying (altering) a DNA sequence which encodes the native polypeptide and amplifying the DNA sequence with suitable means, e.g., by PCR technique. These mutations of the nucleic acids may be generated by either random mutagenesis techniques, such as those techniques employing chemical mutagens, or by site-specific mutagenesis employing oligonucleotides. These nucleic acids conferring substantially the same function, as described above, in substantially the same manner as the exemplified nucleic acids are also encompassed within the present invention.

Accordingly, derivatives of a polypeptide according to the invention (as described above) encoded by the nucleic acids of the invention may also be induced by alterations of the nucleic acids which encodes these proteins.

One of the most widely employed technique for altering a nucleic acid sequence is by way of oligonucleotide-directed site-specific mutagenesis (see Comack B, CURRENT PROTOCOLS IN MOLECULAR BIOLOGY, 8.01-8.5.9, Ausubel F, et al., eds. 1991). In this technique an oligonucleotide, whose sequence contains a mutation of interest, is synthesized as described supra. This oligonucleotide is then hybridized to a template containing the wild-type nucleic acid sequence. In a preferred embodiment of this technique, the template is a single-stranded template. Particularly preferred are plasmids which contain regions such as the f1 intergenic region. This region allows the generation of single-stranded templates when a helper phage is added to the culture harboring the phagemid After annealing of the oligonucleotide to the template, a DNA-dependent DNA polymerase is used to synthesize the second strand from the oliognucleotide, complementary to the template DNA. The resulting product is a heteroduplex molecule containing a mismatch due to the mutation in the oligonucleotide. After DNA replication by the host cell a mixture of two types of plasmid are present, the wild-type and the newly constructed mutant. This technique permits the introduction of convenient restriction sites such that the coding nucleic acid sequence may be placed immediately adjacent to whichever transcriptional or translational regulatory elements are employed by the practitioner.

The construction protocols utilized for *E*. *coli* can be followed to construct analogous vectors for other organisms, merely by substituting, if necessary, the appropriate regulatory elements using techniques well known to skilled artisans.

The isolation of such nucleic acid functional fragments or functional derivatives (as described below) can be carried out by using standard methods as screening methods (e.g., screening of a genomic DNA library) followed by sequencing or hybridisation (with a suitable probe, e.g., derived by generating an oligonucleotide of desired sequence of the "target" nucleic acid) and purification procedures, if appropriate.

The invention also relates to isolated nucleic acids. An "isolated" nucleic acid molecule or nucleotide sequence is intended to mean a nucleic acid molecule or nucleotide sequence which is not flanked by nucleotide sequences which normally flank the gene or nucleotide sequence (as in genomic sequences) and/or has been completely or partially purified from other nucleic acids (e.g., as in an DNA or RNA library). For example, an isolated nucleic acid of the invention may be substantially isolated with respect to the complex cellular milieu in which it naturally occurs. In some instances, the isolated material will form a part of a composition (for example, a crude extract containing other substances), buffer system or reagent mix. In other circumstance, the material may be purified to essential homogeneity, for example as determined by PAGE or column chromatography such as HPLC. This meaning refers correspondingly to an isolated amino acid sequence.

The present invention also encompasses gene products of the nucleic acids of the invention coding for a polypeptide of the invention or a functional fragment or functional derivative thereof. Preferably the gene product codes for a polypeptide according to one of the amino acid sequences of Figure 17 to 24. Also included are alleles, derivatives or fragments of such gene products.

"Gene product" according to the invention relates not only to the transcripts, accordingly RNA, preferably mRNA, but also to polypeptides or proteins, particularly, in purified form.

"Derivatives" or "fragments" of a gene product are defined corresponding to the definitions or derivatives or fragments of the polypeptide or nucleic acid according to the invention.

The invention also provides a vector comprising the nucleic acid of the invention. The terms "construct", "recombinant construct" and "vector" are intended to have the same meaning and define a nucleotide sequence which comprises beside other sequences one or more nucleic acid sequences (or functional fragments, functional derivatifes thereof) of the invention. A vector can be used, upon transformation into an appropriate host cell, to cause expression of the nucleic acid. The vector may be a plasmid, a phage particle or simply a potential genomic insert. Once transformed into a suitable host, the vector may replicate and function independently of the host genome, or may, under suitable conditions, integrate into the genome itself. Preferred vectors according to the invention are *E.coli* XL-Blue MRF' and pBK-CMV plasmid.

The aforementioned term "other sequences" of a vector relates to the following: In general, a suitable vector includes an origin of replication, for example, Ori p, colEl Ori, sequences which allow the inserted nucleic acid to be expressed (transcribed and/or translated) and/or a selectable genetic marker including, e.g., a gene coding for a fluorescence protein, like GFP, genes which confer resistance to antibiotics such as the p-lactamase gene from Tn3, the kanamycin-resistance gene from Tn903 or the chloramphenicol-resistance gene from Tn9.

The term "plasmid" means an extrachromosomal usually self-replating genetic element. Plasmids are generally designated by a lower "p" preceded and/or followed by letters and numbers. The starting plasmids herein are either commercially available, publicly available on an unrestricted basis or can be constructed from available plasmids in accordance with the published procedures. In addition, equivalent plasmids to those described are known to a person skilled in the art. The starting plasmid employed to prepare a vector of the present invention may be isolated, for example, from the appropriate *E. coli* containing these plasmids using standard procedures such as cesium chloride DNA isolation.

A vector according to the invention also relates to a (recombinant) DNA cloning vector as well as to a (recombinant) expression vector. A DNA cloning vector refers to an autonomously replicating agent, including, but not limited to, plasmids and phages, comprising a DNA molecule to which one or more additional nucleic acids of the invention have been added. An expression vector relates to any DNA cloning vector recombinant construct comprising a nucleic acid sequence of the invention operably linked to a suitable control sequence capable of effecting the expression and to control the transciption of the inserted nucleic acid of the invention in a suitable host. Also, the plasmids of the present invention may be readily modified to construct expression vectors that produce the polypeptides of the invention in a variety of organisms, including, for example, *E. coli*, Sf9 (as host for baculovirus), *Spodoptera* and *Saccharomyces*. The literature contains techniques for constructing AV12 expression vectors and for transforming AV12 host cells. U.S. Pat. No. 4,992,373, herein incorporated by reference, is one of many references describing these techniques.

"Operably linked" means that the nucleic acid sequence is linked to a control sequence in a manner which allows expression (e. g., transcription and/or translation) of the nucleic acid sequence.

"Transcription" means the process whereby information contained in a nucleic acid sequence of DNA is transferred to complementary RNA sequence

"Control sequences" are well known in the art and are selected to express the nucleic acid of the invention and to control the transcription. Such control sequences include, but are not limited to a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art and are described, for example, in Goeddel (1990), Gene Expression Technology:Methods in Enzymology 185, Academic Press, San Diego, CA.

Especially a high number of different promoters for different organism is known. For example, a preferred promoter for vectors used in *Bacillus subtilis* is the AprE promoter; a preferred promoter used in *E*. *coli* is the T7/Lac promoter, a preferred promoter used in *Saccharomyces oerevisiae* is PGK1, a preferred promoter used in *Aspergillus niger* is glaA, and a preferred promoter used in *Trichoderma reesei (reesei)* is cbhI. Promoters suitable for use with prokaryotic hosts also include the beta-lactamase (vector pGX2907 (ATCC 39344) containing the replicon and beta-lactamase gene) and lactose promoter systems (Chang et al. (1978), Nature (London), 275:615; Goeddel et aL (1979), Nature (London), 281:544), alkaline phosphatase, the tryptophan (trp) promoter system (vector pATH1 (ATCC 37695) designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable a person skilled in the art to ligate them to DNA encoding the polypeptides of the instant invention using linkers or adapters to supply any required restriction sites. Promoters for use in bacterial systems also will contain a Shine-Dalgarno sequence operably linked to the DNA encoding the desired polypeptides.

Useful expression vectors, for example, may consist of segments of chromosomal, non-chromosomal and synthetic DNA sequences such as various known derivatives of SV40 and known bacterial plasmids, e.g., plasmids from *E*. *coli* including col E1, pBK, pCR1, pBR322, pMb9, pUC 19 and their derivatives, wider host range plasmids, e.g., RP4, phage DNAs e.g., the numerous derivatives of phage lambda, e.g., NM989, and other DNA phages, e.g., M13 and filamentous single stranded DNA phages, yeast plasmids, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors of the present invention are known in the art and are described generally in, for example, Sambrook J, Maniatis T (1989) *supra.*

The invention also provides a host cell comprising a vector or a nucleic acid (or a functional fragment, or a functional derivative thereof) according to the invention.

"Host cell" means a cell which has the capacity to act as a host and expression vehicle for a nucleic acid or a vector according to the present invention. The host cell can be e.g., a prokaryotic, an eukaryotic or an archaeon cell. Host cells comprising (for example, as a result of transformation, transfection or tranduction) a vector or nucleic acid as described herein include, but are not limited to, bacterial cells (e.g., *R. marinus, E. coli, Streptomyces, Pseudomonas, Bacillus, Serratia marcescens, Salmonella typhimurium*), fungi including yeasts (e. g., *Saccharomycies cerevisie, Pichia pastoris*) and molds (e.g., *Aspergillus sp*.), insect cells (e.g., Sf9) or mammalian cells (e.g., COS, CHO). In a preferred embodiment according to the present invention, host cell means the cells of *E. coli*.

Eukaryotic host cells are not limited to use in a particular eukaryotic host cell. A variety of eukaryotic host cells are available, e.g., from depositories such as the American Type Culture Collection (ATCC) and are suitable for use with the vectors of the present invention. The choice of a particular host cell depends to some extent on the particular expression vector used to drive expression of the nucleic acids of the present invention. Eukaryotic host cells include mammalian cells as well as yeast cells.

The imperfect fungus *Saccharomyces oerevisiae* is the most commonly used eukaryotic microorganism, although a number of other strains are commonly available. For expression in *Saccharomyces sp*., the plasmid YRp7 (ATCC-40053), for example, is commonly used (see. e,g., Stinchcomb L. et al. (1979) Nature, 282:39; Kingsman J. al. (1979), Gene, 7:141; S. Tschemper et al. (1980), Gene, 10:157). This plasmid already contains the trp gene which provides a selectable marker for a mutant strain of yeast lacking the ability to grow in tryptophan.

Suitable promoting sequences for use with yeast hosts include the promoters for 3-phosphoglycerate kinase (found on plasmid pAP12BD (ATCC 53231) and described in U.S.

Pat. No. 4,935,350, issued Jun. 19, 1990, herein incorporated by reference) or other glycolytic enzymes such as enolase (found on plasmid pAC1 (ATCC 39532)), glyceraldehyde-3-phosphate dehydrogenase (derived from plasmid pHcGAPC1 (ATCC 57090, 57091)), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase, as well as the alcohol dehydrogenase and pyruvate decarboxylase genes of Zymomonas mobilis (U.S. Pat. No. 5,000,000 issued Mar. 19, 1991, herein incorporated by reference).

Other yeast promoters, which are inducible promoters, having the additional advantage of their transcription being controllable by varying growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV (ATCC 39475) and described in U.S. Pat. No. 4,840,896, herein incorporated by reference), glyceraldehyde 3-phosphate dehydrogenase, and enzymes responsible for maltose and galactose (e.g. GAL1 found on plasmid pRY121 (ATCC 37658)) utilization. Yeast enhancers such as the UAS Ga1 from Saccharomyces cerevisiae (found in conjuction with the CYC1 promoter on plasmid YEpsec--hI1beta ATCC 67024), also are advantageously used with yeast promoters.

A vector can be introduced into a host cell using any suitable method (e.g., transformation, electroporation, transfection using calcium chloride, rubidium chloride, calcium phosphate, DEAEdextran or other substances, microprojectile bombardment, lipofection, infection or transduction). Transformation relates to the introduction of DNA into an organism so that the DNA is replicable, either as an extrachromosomal element or by chromosomal integration. Methods of transforming bacterial and eukaryotic hosts are well known in the art. Numerous methods, such as nuclear injection, protoplast fusion or by calcium treatment are summerized in SambrookJ, Maniatis T (1989) *supra*. Transfection refers to the taking up of a vector by a host cell whether or not any coding sequences are in fact expressed. Successful transfection is generally recognized when any indication or the operation or this vector occurs within the host cell.

Another embodiment of the invention provides a method for the production of the polypeptide of the invention comprising the following steps:
(a) cultivating a host cell of the invention and expressing the nucleic acid under suitable conditions;
(b) isolating the polypeptide with suitable means.

The polypeptides according to the present invention may also be produced by recombinant methods. Recombinant methods are preferred if a high yield is desired. A general method for the construction of any desired DNA sequence is provided, e.g., in Brown J. et al. (1979), Methods in Enzymology, 68:109; Sambrook J, Maniatis T (1989), *supra*.

According to the invention an activity-based screening in metagenome library was used as a powerful technique to isolated new enzymes from the big diversity of microorganisms found in rumen ecosystem (Lorenz, P et al. (2002) Screening for novel enzymes for biocatalytical processes:accessing the metagenome as a resource of novel functional sequence space, Current Opinion in Biotechnology 13:572-577). For this purposes an activity selection technique with Ostazin-brilliant red-hydroxyethyl cellulose as assay substrate, was used. This technology enable screening of 105 -109 clones/day from genomes of between 1 and 15,000 microorganisms. In detail, this method is described in the Examples below.

The polypeptide can be isolated from the culture medium by conventional procedures including separating the cells from the medium by centrifugation or filtration, if necessary after disruption of the cells, precipitating the proteinaceous components of the supernatant or filtrate by means of a salt, e. g., ammonium sulfate, followed by purification by a variety of chromatographic procedures, e. g., ion exchange chromatography, affinity chromatography or similar art recognized procedures.

Efficient methods for isolating the polypeptide according to the present invention also include to utilize genetic engineering techniques by transforming a suitable host cell with a nucleic acid or a vector provided herein which encodes the polypeptide and cultivating the resultant recombinant microorganism, preferably *E.coli*, under conditions suitable for host cell growth and nucleic acid expression, e.g., in the presence of inducer, suitable media supplemented with appropriate salts, growth factors, antibiotic, nutritional supplements, etc.), whereby the nucleic acid is expressed and the encoded polypeptide is produced.

In additional embodiments the polypeptide of the invention can be produced by *in vitro* translation of a nucleic acid that encodes the polypeptide, by chemical synthesis (e. g., solid phase peptide synthesis) or by any other suitable method.

Skilled artisans will recognize that the polypeptides of the present invention can also be produced by a number of different methods. All of the amino acid sequences of the invention can be made by chemical methods well known in the art, including solid phase peptide synthesis, or recombinant methods. Both methods are described in U.S. Pat. No. 4,617,149, the entirety of which is herein incorporated by reference.

The principles of solid phase chemical synthesis of polypeptides are well known in the art and are described by, e.g., Dugas H. and Penney C. (1981), Bioorganic Chemistry, pages 54-92. For examples, peptides may be synthesized by solid-phase methodology utilizing an Applied Biosystems 430A peptide synthesizer (commercially available from Applied Biosystems, Foster City, Calif.) and synthesis cycles supplied by Applied Biosystems. Protected amino acids, such as t-butoxycarbonyl-protected amino acids, and other reagents are commercially available from many chemical supply houses.

Sequential t-butoxycarbonyl chemistry using double couple protocols are applied to the starting p-methyl benzhydryl amine resins for the production of C-terminal carboxamides. For the production of C-terminal acids, the corresponding pyridine-2-aldoxime methiodide resin is used. Asparagine, glutamine, and arginine are coupled using preformed hydroxy benzotriazole esters. The following side chain protection may be used:
Arg, Tosyl
Asp, cyclohexyl
Glu, cyclohexyl
Ser, Benzyl
Thr, Benzyl
Tyr, 4-bromo carbobenzoxy

Removal of the t-butoxycarbonyl moiety (deprotection) may be accomplished with trifluoroacetic acid (TFA) in methylene chloride. Following completion of the synthesis the peptides may be deprotected and cleaved from the resin with anhydrous hydrogen fluoride containing 10% meta-cresol. Cleavage of the side chain protecting group(s) and of the peptide from the resin is carried out at zero degrees centigrade or below, preferably -20°C. for thirty minutes followed by thirty minutes at 0 °C.

After removal of the hydrogen fluoride, the peptide/resin is washed with ether, and the peptide extracted with glacial acetic acid and then lyophilized. Purification is accomplished by size-exclusion chromatography on a Sephadex G-10 (Pharmacia) column in 10% acetic acid

Another embodiment of the invention relates to the use of the polypeptide, the nucleic acid, the vector and/or the host cell of the invention (hereinafter "substances of the invention") for the treatment of cellulosic textiles or fabrics, e.g. as an indegrient in compositions, preferably detergent compositions or fabric softener compositions. Consequently, the invention relates also to detergent compositions including a polypeptide according to the invention.

Cellulases and thus substances of the invention can be used for modification of a broad range of cellulose containing textile material. It opens also the possibility to make use of cellulase technology for introduction of subtle decorative patterns on dyed cloth. This result in more bright colors at those treated places and thus subtle color differences following the pattern. Examples of the uses of substances of the invention can also be found in the denim garment washing industry to give a stone washed look to denim without the use of pumice stones and in the textile finishing industry to give the cloth a smooth surface with improved wash and wear resistance and a softer hand (biopolishing).

The treatment of cellulosic textiles or fabrics includes textile processing or cleaning with a composition comprising a polypeptide of to the present invention. Such treatments include, but are not limited to, stonewashing, modifying the texture, feel and/or appearance of cellulose containing fabrics or other techniques used during manufacturing or cleaning/reconditioning of cellulose containing fabrics. Additionally, treating within the context of this invention contemplates the removal of immature cotton from cellulosic fabrics or fibers.

The detergent resistance or in other words the effect of surfactants on the activity of the polypeptide of the invention was measrued. The results are given in Figure 3 and show that the activity of the polypeptide is increased in the presence of Triton X-100 and SDS whereas other surfactants, like Tween 20-80, polyethylene alkyl ether did not affect the activity of the polypeptides Also, the effect of several commonly used inhibitors on the activity of the polypeptide of the invention was investigated. Sulfhydryl inhibitors such as N-ethylmaleimide, iodoacetate and *p*-chloromercuribenzoate were all inhibitory. The chelating agents EDTA (ethylenediaminetetraacetic acid) and EGTA (ethyleneglycoltetraacetic acid) a more efficient chelator of Ca²⁺ (inhibition > 30%) and O-phenanthroline (inhibition > 71%) had essentially no effect on the activity of the polypeptides or were slightly stimulatory. In summary, the polypeptides of the invention are well suited to be used for the treatment of cellulosic textiles or fabricss, especially as an ingredient in a detergent composition, because ot their high activity at high pH and high stability against metal ions or various components of laundry products such as surfactants, chelating agents or proteinases. In general, they are also suited to be used in compositons containing several commonly used inhibitors.

Thus, substances of the present invention can be employed in a detergent composition. Such a detergent compositions is useful as pre-wash compositions, pre-soak compositions, or for cleaning during the regular wash or rinse cycle. Preferably, the detergent compositions of the present invention comprise an effective amount of a substance of the invention, surfactants, builders, electrolytes, alkalis, antiredeposition agents, bleaching agents, antioxidants, solubilizer and other suitable ingredients known in the art.

An "effective amount" of polypeptide employed in the detergent compositions of this invention is an amount sufficient to impart the desirable effects and will depend on the extent to which the detergent will be diluted upon addition to water so as to form a wash solution.

"Surfactants" of the detergent composition can be anionic (e.g., linear or branche alkylbenzenesulfonates, alkyl or alkenyl ether sulfates having linear or branche alkyl groups or alkenyl groups, alkyl or alkenyl sulfates, olefinsulfonates and alkanesulfonates), ampholytic (e.g., quaternary ammonium salt sulfonates and betaine-type ampholytic surfactants) or non-ionic surfactants (e.g., polyoxyalkylene ethers, higher fatty acid alkanolamides or alkylene oxide adduct thereof, fatty acid glycerine monoesters). It is also possible to use mixtures of such surfactants.

"Builders" of the detergent composition include, but are not limited to alkali metal salts and alkanolamine salts of the following compounds: phosphates, phosphonates, phosphonocarboxylates, salts of amino acids, aminopolyacetates high molecular electrolytes, non-dissociating polymers, salts of dicarboxylic acids, and aluminosilicate salts.

"Electrolytes" or "alkalis" of the detergent composition include, for example, silicates, carbonates and sulfates as well as organic alkalis such as triethanolamine, diethanolamine, monoethanolamine and triisopropanolamine.

"Antiredeposition agents" of the detergent composition include, for example, polyethylene glycol, polyvinyl alcool, polyvinylpyrrolidone and carboxymethylcellulose.

"Bleaching agents" of the detergent composition include, for example, potassium monopersulfate, sodium percarbonate, sodium perborate, sodium sulfate/hydrogen peroxide adduct and sodium chloride/hydrogen peroxide adduct or/and a photo-sensitive bleaching dye such as zinc or aluminum salt of sulfonated phthalocyanine further improves the detergenting effects.

"Antioxidants" of the detergent composition include, for example, tert-butyl-hydroxytoluene, 4,4'-butylidenebis (6tert-butyl-3-methylphenol), 2,2'-butylidenebis (6-tert-butyl-4-methylphenol), monostyrenated cresol, distyrenated cresol, monostyrenated phenol, distyrenated phenol and 1,1-bis (4hydroxy-phenyl) cyclohexane.

"Solubilizer" of the detergent composition include, for example, lower alcohols (e.g., ethanol), benzenesulfonate salts, lower alkylbenzenesulfonate salts (e.g., p-toluenesulfonate salts), glycols (e.g., propylene glycol), acetylbenzene-sulfonate salts, acetamides, pyridinedicarboxylic acid amides, benzoate salts and urea.

The detergent compositions of the present invention may be in any suitable form, for example, as a liquid, in granules, in mulsions, in gels, or in pastes. Such forms are well known in the art and are described e.g., in U.S. Patent No. 5,254,283 which is incorporated herein by reference in its entirety.

Further embodiments of the invention of the invention relate to the use of the substances of the invention in the treatment of pulp and paper industry, in consumer products, particularly food products and as an additive for animal food for purposes known in the art,.

For example, cellulases, and therefore polypeptides of the invention, are known improve the drainability of wood pulp or paper pulp, to increase the value of animal food, enhance food products and reduce fiber in grain during the grain wet milling process or dry milling process.

Thus, substances of the invention can be applied in bakery to improve, for example, bread volume. Supplementation of polypeptides of the invention is useful to increase the total tract digestibility of organic matter and fiber during animal feeding. The proportion of the diet to which substances of the invention are applied must be maximized to ensure a beneficial response. For example, it is known that small proportion of cellulases on the diet increase the microbial N synthesis for cows.

Furthermore, substances of the invention can be applied to improve the use of cellulosic biomass and may offer a wide range of novel applications in research, medicine and industry. One exmaple is the production of biodiesel. Cellulases (and thus, substances of the invention) are able to hydrolyse cellulolytic substrate to more hydrolysable sugar which can be used as raw materil for ethanol production. Related to medicine they could be applied for the destruction of pathogenic biofilm which are protected for the environment though a polysaccharide cover. They could help for its degradation, making them more accessible for antibiotic treatment. Cellulases (and thus, substances of the invention) are useful as a possible alternative to the current practice of open air burning of sugarcane residue by farmers. They are also useful as a chiral stationary phase in liquid chromatographic separations of enantiomers. Cellulases (and thus, substances of the invention) are also useful in the synthesis of trasnglycosilation product using cellulolytic substrate as raw materials. The trasnglycosylated product can be use for their beneficial effect in health as food additive, similar to FOS and GOS (probiotics).

Another embodiments of the invention relates to the use of the substances of the invention as a protoplast preparation agent, a herbicide or a drench in ruminants. Yet another embodiment of the invention relates to the use of the substances of the invention in the production of anti-cellulase reagents, e.g., bacteriocide and fungicide.

The substances of the invention can be expressed, for example, in plants.

The treatment according to the invention also comprises preparing an aqueous solution which contains an effective amount of the polypeptide of the invention together with other optional ingredients, for example, a surfactant, as described above, a scouring agent and/or a buffer. A buffer can be employed to maintain the pH of the aqueous solution within the desired range. Such suitable buffers are well known in the art. As described above, an effective amount of the polypeptide will depend on the intended purpose of the aqueous solution.

The following figures and examples are thought to illustrate the invention and should not be constructed to limit the scope of the invention thereon. All references cited by the disclosure of the present application are hereby incorporated in their entirety by reference.

### Figures

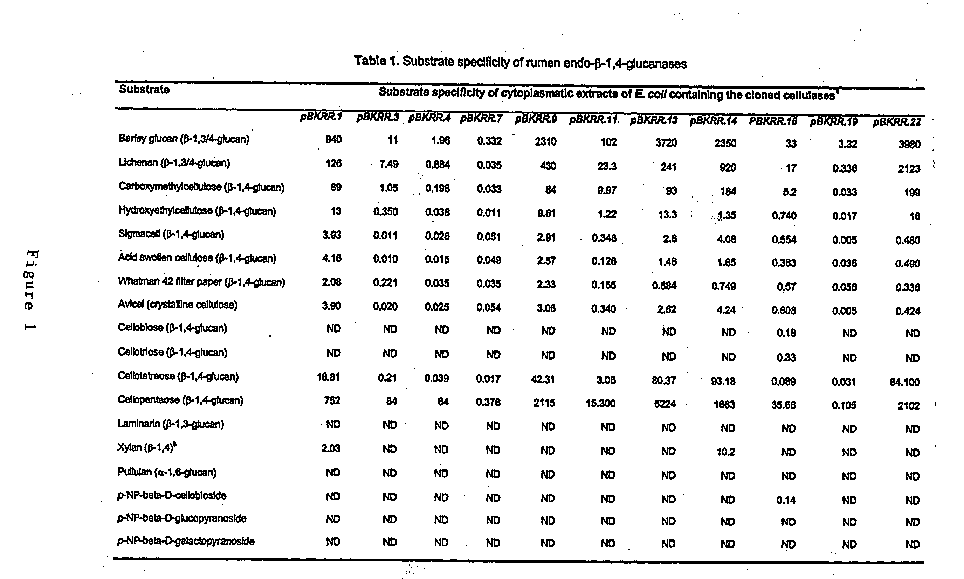

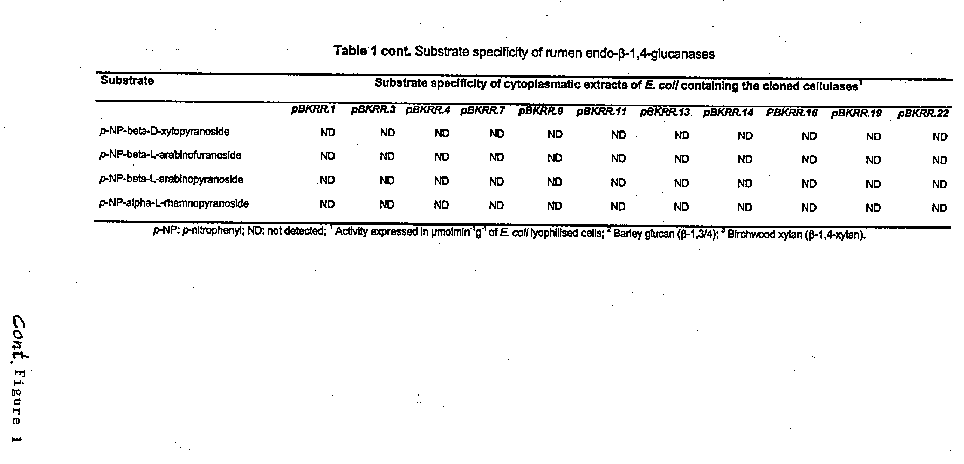

**Figure 1** shows **Table 1** representing substrate specifity of rumen endo-beta-1,4-glucanases for various substrates. The values for clones pBKRR.1, 3, 4, 7, 9, 11, 13, 14, 16, 19 and 22 are depicted. The activity is expressed in µmol min⁻¹g⁻¹ of *E. coli* lyophilised cells.

Endoglucanases were expressed in *Escherichia coli* under the control of P_{lac}-promoter. The relative level of cellulase expression was found to be pBKRR.22 > pBKRR.14 > pBKRR.1 > pBKRR9 > pBKRR.13 > pBKRR.3 > pBKRR.4 > pBKRR.7 > pBKRR.11 > pBKRR.16, and was in the range from 5 to 0.4% (w/w of total proteins). A preliminary quantitative assessment of enzymatic activities was performed by testing *E. coli* lyophilized cells bearing cellulose cDNAs, for their ability to hydrolyze various cellulose substrates (4% wt/vol), including glucan oligosaccharides with beta-1,4 backbone linkage (cellobiose, cellotriose, cellotetrose, cellopentose) or beta-1,3 linkage (laminarin), glucan polysaccharides, i.e. Barley glucan (beta-1,3/4), Lichenan (beta-1,3/4) and carboxymethyl cellulose (CMC), hydroxyethyl cellulose, Sigmacell®, acid swollen cellulose or filter paper (beta-1,4) as well as Avicel (crystalline cellulose), birchwood xylan (beta -1,4), and solubles *p*-nitrophenyl derivatives.

Results show that *E. coli* extracts expressing cellulose cDNAs had the highest activity towards cellulose oligosaccharides, specially (1,3),(1,4)-beta-glucans (from 0.3 to 3980 µmol min⁻¹ g⁻¹ of *E. coli* cells), such as barley beta-glucan and lichenan, similar to other endoglucanases found in 12 different families (Bauer et al., 1999, An Endoglucanase, EglA, from the Hyperthermophilic Archaeon *Pyrococcus furiosus* hydrolyzes beta-1,4 Bonds in Mixed-Linkage (1→3),(1→4)-beta-D-Glucans and Cellulose *J. Bacteriol*., 181: 284-290). pBKRR.1, pBKRR.9, pBKRR.13, pBKRR.14 and pBKRR.22 had also significant activity towards substituted cellulose-based substrates, with only beta-1,4 linkages, such as CMC (from 89 to 199 µmol min⁻¹ g⁻¹ of lyophilized cells). pBKRR.11 and pBKRR.16 (from 5.2 to 9.9 µmol min⁻¹ g⁻¹), and in less extension pBKRR.3, pBKRR.4, pBKRR.7 and pBKRR.19 (from 0.19 to 1.05 µmol min⁻¹ g⁻¹) were able to hydrolyze CMC. pBKRR16 hydrolyzed to a certain extent cellobiose and cellotriose as well as *p*-nitrophenyl cellobiose. Cellotetrose and cellopentose were hydrolyzed with all the enzymes to a high extent. Reducing sugars were released from Sigmacell, acid-swollen cellulose and filter paper, although the activity was only 0.2 to 4.0% of that shown against CMC. Since all enzymes are much more active on beta-glucans, carboxymethyl and hydroxyethyl cellulose, which gives more adjacent glucosyl residues in the cellulose backbone comparing with Sigmacell or filter paper, it is considered as more appropriate to refer to these enzymes as endo-beta-1,4-glucanases. This agrees also with the unability to hydrolyze soluble *p*-nitrophenyl derivatives. Xylanase activity was found in cell extracts expressing pBKRR.1 and pBKRR.14, although the activity was 20-40 times lower than that found with CMC.

In summary, it is concluded that: i) pBKRR1 and pBKRR14 have hallmark qualities of an endoglucanase which act mainly on beta-1,4 -glucans but are also capable of hydrolyzing xylan, ii) pBKRR16 have both endoglucanase and cellobiohydrolase activity, and iii) pBKRR.3, 4, 7, 9, 11, 13, 19 and 22 are endo-beta-1,4 -glucanases.

**Figure 2** represents **Table 2** which contains the data obtained by purification of endoglucanases of seven clones (pBKRR.1, 9, 11, 13, 14, 16 and 22) with respect to the specific activity. Said clones were chosen, because they exhibit highest activity on the cellulose substrates. They were purified to homogeneity and their specific activity was estimated at pH 5.6 and 50°C, using CMC as substrate.

The specific activity is expressed in µmol min⁻¹ mg⁻¹ and gives the following values: 40.56 (µmol min⁻¹ mg⁻¹) for pBKRR.1, 41.59 for pBKRR.9, 6.42 for pBKRR.11, 65.00 for pBKRR.13, 34.60 for pBKRR.14, 4.12 for pBKRR.16 and 68.30 for pBKRR.22.

The average value of endoglucanase activity of the tested enzymes according to the invention was 4.5, 14, 2, 1.1 and 19 times higher than the activity of endoglucanases known in the art and isolated from hyperthermophilic archaeon *Pyrococcus furiosus* (Bauer et aL, 1999), anaerobic fungus *Orpinomyces joyonii* strain SG4 (Qiu et al., 2000), two *Bacillus* strains, CH43 and HR68 (Mawadza et al., 2000), thermophilic fungus *Thermoascus aurantiacus* (Parry et al., 2002) and filamentous fungus *Aspergillus niger* (Hasper et aL, 2002), respectively.

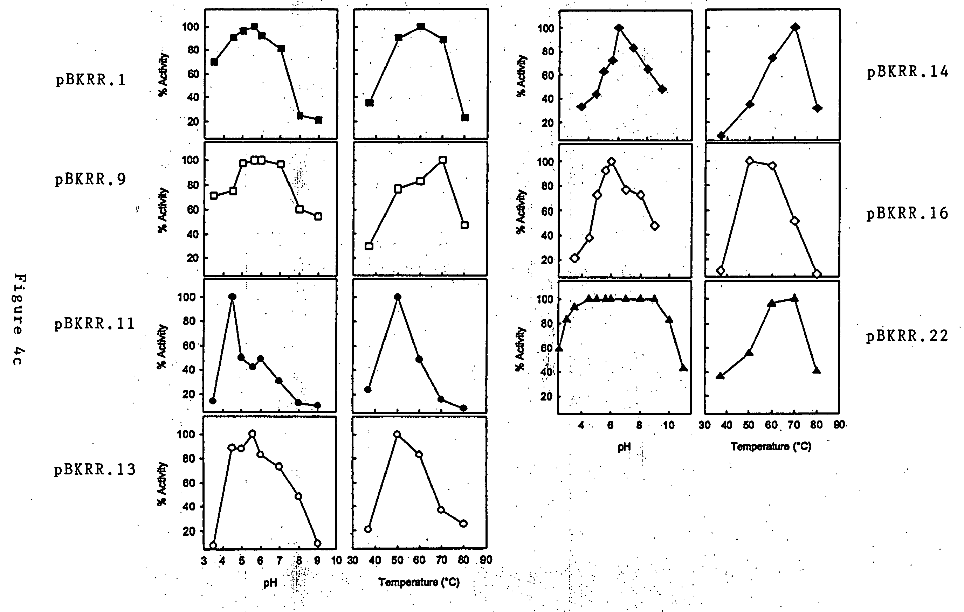

**Figure 3** represents **Table 3** giving an overview over the biochemical properties of the cellulases according to the invention. Reaction was performed at the optimum pH and temperature for each clone using CMC as substrate. The pH and temperature used in the reactions are shown in Figures 4 a-c. In detail, cell lysates from clones possessing higher activity endoglucanases were tested for their ability to hydrolyze CMC under pH conditions ranging from 3.5 to 10.5 at a temperature of 40°C. The values for clones pBKRR.1, 9, 11, 13, 14, 16 and 22 are depicted. For each clone the following data are given:
- maximum pH at which the remaining activity after 24 h incubation at the indicate pH is > 90% (column "Stable at pH"). It can be seen that pBKRR.22 showed the greatest activity-pH profile and showed activity at both alkaline (pH 9.0-10.0) and acidophilic (3.5-4.0) conditions without variation of enzyme activity. pBKRR.1 and pBKRR.9 were more active at pH 5.6, although they retained 65-80% of their activity between 3.5 and 7.0. pBKRR.11 showed maximal activity at pH 4.5 and its activity decreased at lower or higher pH. pBKRR.13 was active and stable at pH from 4.5 to 6.0 and pBKRR.14 and pBKRR.16 were more active at 5.6-6.0 and lost > 60% of their activity at pH values of > 2.
- maximum temperature at which the remaining activity after 2 h incubation is > 90% (column "Stable at Temperature"). Thermal activity and thermostability was determined at the pH optima. pBKRR.9, pBKRR.14 and pBKRR.22 exhibited improved activity and thermostability at 70°C, pBKRR.1 and pBKRR.16 at 60°C, whereas pBKRR.11 and pBKRR.13 were more active at 50°C, losing > 60% of their activity at higher temperatures. All cellulases exhibited calcium-independent thermostability except pBKRR.13 which exhibited greater thermostability at 60-70°C in the presence of 4-10 mM Ca²⁺ compared with that shown in absence of this cation (Top temperature: 50°C).
- specific activity in µmol min⁻¹ g⁻¹ of cytoplasmatic extracts of *E. coli* containing the cloned cellulase genes,
- solvent resistance given by remaining activity in a mixture buffer: DMSO (4:1). Activity was measured after 2 h incubation and compared with a control reaction in buffer lacking dimethylsulfoxide,
- detergent resistance given by the remaining activity in the corresponding buffer (optimum pH) containing the indicate detergent at a concentration for 0 to 3%. Activity was measured after 2 h incubation and compared with a control reaction in buffer lacking detergent. pBKRR.1 and pBKRR.13 were 150 and 214% more active and stable at pH 5.6 and 70°C or pH 5.6 and 50°C, respectively, in the presence of Triton X-100 (1-3%) but not in the presence of SDS (1 mM). PBKRR.9 was 184% and 108% more active and stable by 1-3% Triton X-100 and SDS at pH 4.5 and 70°C. Similar result was obtained for pBKRR.14 which was 134 and 161% more active in the presence of Triton X-100 and SDS (1-3%). PBKRR.22 was resistant to Triton X-100 up to 1-3% but was inhibited by SDS at concentrations higher than 1 mM. Only pBKRR.11 and pBKRR.16 showed medium stability in the presence of both surfactants (62-50% of the maximal activity). Other surfactants, such as Tween 20-80, polyoxyethylene alkyl ether or alkyl sulphate ,- sulfonate, did not affect the activity of all the endoglucanases up to 3% w/v.
- cation dependence: all indicated cations were added as chloride salts and tested at a concentration of 10 to 100 mM. As described above, all cellulases exihited calcium independent thermostability except pBKRR.13. The effect of mono- (Na⁺, K⁺, NH4⁺), and divalent cations (Ca ²⁺, Mn²⁺, Mg²⁺, Sr²⁺, Fe²⁺, Cu²⁺, Ni²⁺, Co²⁺, Zn²⁺) was first examined by treating the enzyme with chelating agents (EDTA and EGTA) to remove any endogenous divalent cations, followed by adding cations to the reaction mixture.

The results derived from these experiments showed that pBKRR.1, 11, 14 and 16 were not influenced by the tested cations, although Mg²⁺ showed a moderate stimulatory effect (127%). PBKRR.9 was slightly activated by all tested mono- and divalent cations (102 to 294%), whereas NH₄⁺ and Mg²⁺ partially inhibited the cellulases (10-15%). Interestingly, pBKRR.13 was highly activated (from 193 to 569%) by the majority of mono- and divalent cations including calcium (234%) which was the only clone affected by this cation.

**Figure 4 a-c** represent the results of the determination of pH and temperature optima showing graphically views of the pH-dependence activity and temperature-dependence activity of the polypeptides according to the invention. The pH optima of rumial endoglucanases were measured under conditions described in Example 5: 4% CMC, 0.5 mL of 50 mM buffer at the corresponding pH and 0.1 mg *E. coli* cell lysates, for 5-30 min, at 40°C. Effect of temperature on endoglucanase activity was measured in 50 mM sodium acetate buffer pH 5.6.
**Figure 4a** shows a graphically view of the pH-dependent activity,
**Figure 4b** shows a graphically view of the temperature-dependence activity and
**Figure 4c** shows a graphically view of a comparison of the pH- and temperature-dependent activity at the same conditions. The percentage of the maximal response at each pH and temperature is given as follows: clones of the left side figures: pBKRR.1 → pBKRR:9 → pBKRR.11 → pBKRR.13 (from the top to the bottom); right side figures: pBKRR.14 → pBKRR.16 → pBKRR.22 (from the top to the bottom).

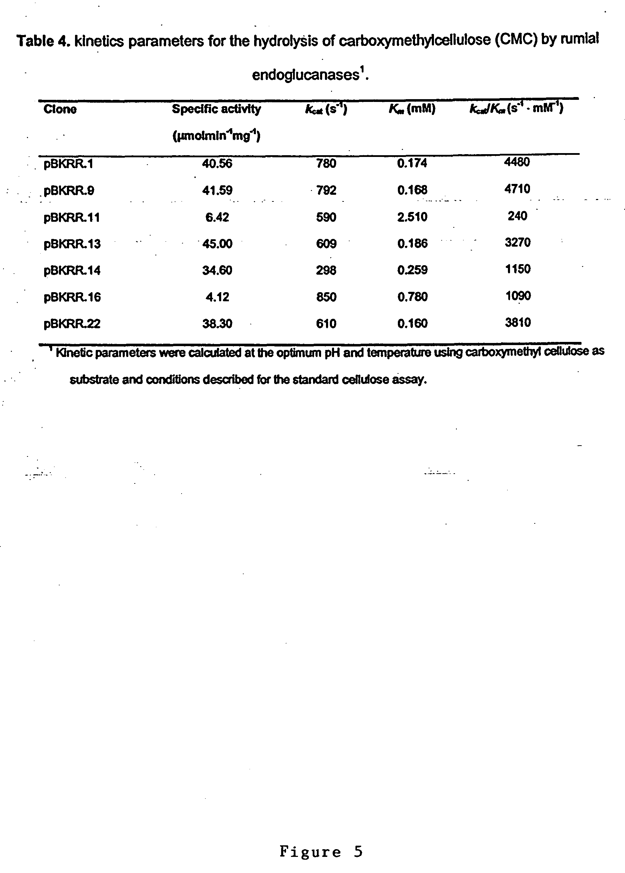

**Figure 5** corresponds to **Table 4** exhibiting kinetic parameters (substrate saturation kinetics) for the hydrolysis of CMC by rumial endoglucanases. Data were calculated at optimal conditions of pH, temperature and Ca²⁺ requirements using CMC as substrate under conditions described in Example 5. The results of clones pBKRR.1, 9, 11, 13, 14, 16 and 22 are given. The saturation curve was hyperbolic (not shown), and the double-reciprocal plot of Lineweaver and Burke (1934) yielded an apparent *k*cat and *Km* as shown. Endoglucanases derived from pBKRR1, pBKRR.9, pBKRR.13, pBKRR.14 and pBKRR.22 had a relatively low *Km* for CMC (from 0.160 to 0.259 mM) and quite high maximum catalytic efficiency (*k*cat/*Km*), which is in the range from 240 to 4710 s⁻¹mM⁻¹. These results confirm that cellulases according to the invention have the highest catalytic efficiency towards CMC yet reported for any endoglucanase, and confirm the high affinity of endo-beta-1,4-glucanases isolated from rumen samples derived from New Zealand dairy cows for cellulose-based substrates.

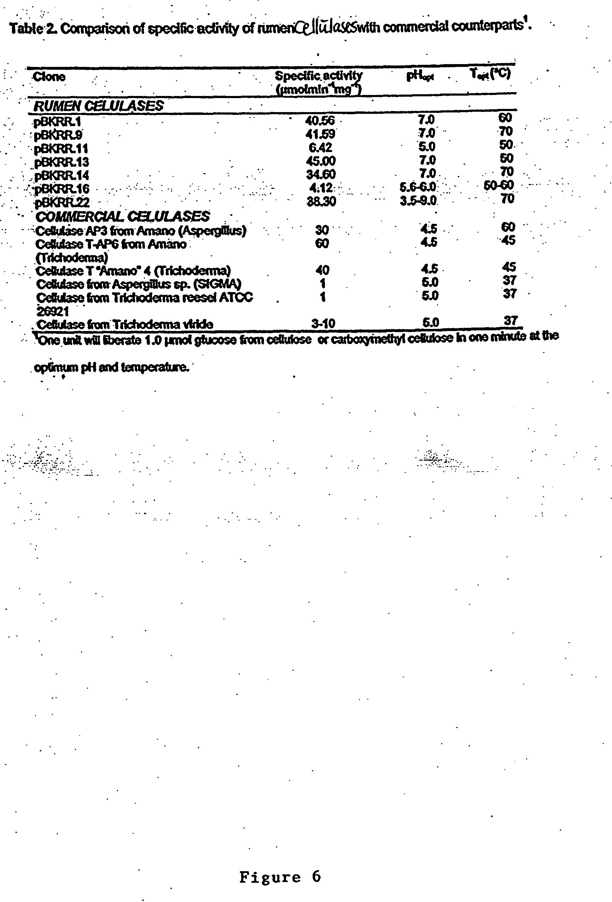

**Figure 6** shows an comparison of specific activity of rumen cellulases with commercial counterparts. The activity is expressed in µmol min⁻¹g⁻¹ of protein. The release of 1.0 µmol glucose from cellulose or CMC per minute at optimum pH corresponds to 1 unit. The results of rumen cellulases of clones pBKRR.1, 9, 11, 13, 14, 16 and 22 according to the invention are given. Results of experiments with commercially available cellulases from *Aspergillus* and *Trichodema* are presented for comparative reasons.

To summerize the results shown in Figure 6, the specific activity as well as the optimum pH and temperature of rumen cellulases are considerably higher than of the commercial cellulases. pBKRR.9 gives unprecedented results with a specific activity of 41.59 µmol min⁻¹ g⁻¹ at pH 7.0 and 70°C and pBKRR.13 with a specific activity of 45.00 µmol min⁻¹ g⁻¹ at pH 7.0 and 50°C followed by pBKRR.1, pBKRR.22 and pBKRR.14. All tested clones of the invention show much better results than the commercial enzymes (best result: a specific activity of 40.00 µmol min⁻¹ g⁻¹ at pH 4.5 and 45°C).

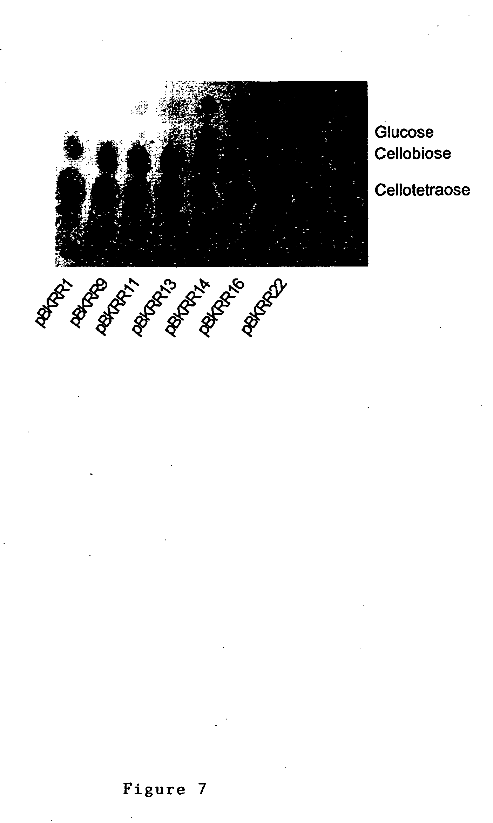

**Figure 7** depicts the result of thin layer chromatography TLC analysis of products of CMC hydrolyzed by endoglucanases derived from a rumial genomic DNA library. The methods used for cellulase and substrate preparation, hydrolysis, TLC, and visualization are described in Example 5. The hydrolysis products glucose, cellobiose, cellotriose are shown on the right side. The amound of end products of CMC hydolysis by the most active cellulases pBKRR1, 9, 11, 13, 14, 16, 22 were determined by TLC. The main hydrolysis products were cellobiose and cellotriose. Only small amounts of glucose were detected using cells expressing pBKRR16. Different pattern was found when using short-chain beta-1,4-glucans. Thus, the presence of cellobiose and traces of glucose were observed during hydrolysis of cellotriose only in the presence of cells expressing pBKRR16. Cellotetrose was cleaved predominantly to cellobiose, whereas cellopentose was mainly hydrolyzed to cellobiose and cellotriose (not shown). Since small amounts of glucose were observed when using cells expressing pBKRR16 for cellotetraose and cellopentaose, pBKRR.16 is able to hydrolize cellobiose and cellotriose.

**Figures 9 to 16** represent the nucleic acid sequences of sixteen positive clones obtained from the genomic library constructed from ruminal ecosystem. In detail
**Figure 9** represents the identical nucleic acid sequence of clones pBKRR 1 and pBKRR 21. This clones contain two cellulases (endoglucanases) so that two nucleic acid sequences are represented in Figure 9, namely RR01-1 (=RR21) and RR01-2 (=RR21).
**Figure 10** represents the identical nucleic acid sequence of clones pBKRR 2 and pBKRR 16. This clones contain two cellulases (endoglucanases) so that two nucleic acid sequences are represented in Figure 10, namely RR02-1 (=RR16) and RR02-2 (=RR16).
**Figure 11** represents the identical nucleic acid sequence of pBKRR 22, pBKRR 6, pBKRR 8 and pBKRR 23.
**Figure 12** represents the nucleic acid sequence of pBKRR 7.
**Figure 13** represents the nucleic acid sequence of pBKRR 9.
**Figure 14** represents the identical nucleic acid sequence of pBKRR 11, pBKRR 10 and pBKRR 12.
**Figure 15** represents the nucleic acid sequence of pBKRR 13 and
**Figure 16** represents the identical nucleic acid sequence of pBKRR 14 and pBKRR 20-1.

**Figures 17 to 24** represent the amino acid sequences corresponding to the nucleic acid sequences of the sixteen positive clones represented in Figures 9 to 16. In detail
**Figure 17** represents the amino acid sequence of pBKRR 1 and pBKRR 21. This clones contain two cellulases (endoglucanases) so that two amino acid sequences are represented in Figure 17, namelyRR01-1 (=RR21) and RR01-2 (=RR21).

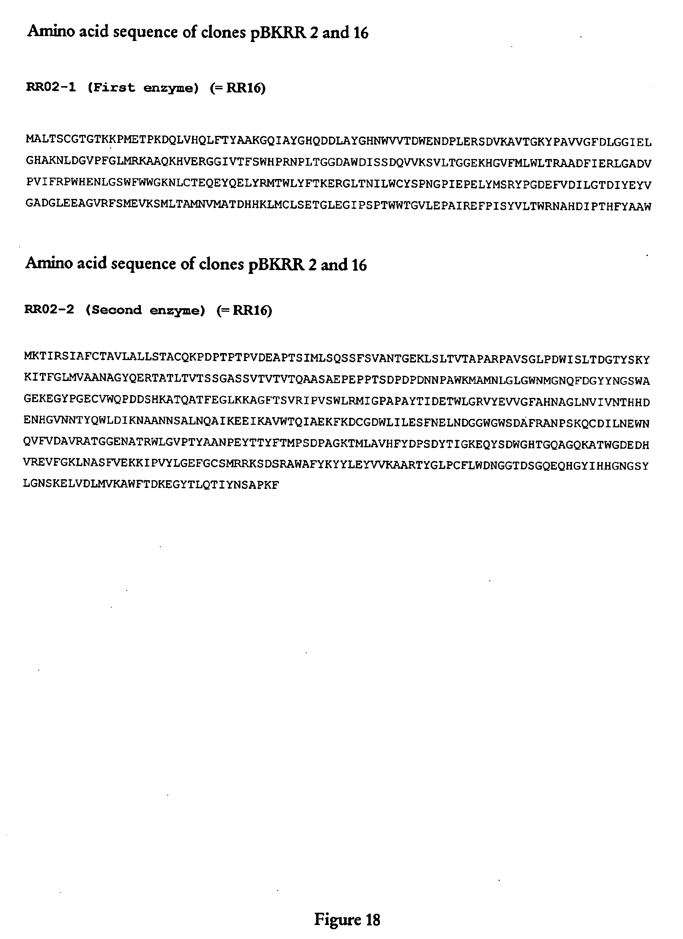

**Figure 18** represents the amino acid sequence of pBKRR 2 and pBKRR 16. This clones contain two cellulases (endoglucanases) so that two amino acid sequences are represented in Figure 18, namely RR02-1 (=RR16) and RR02-2 (=RR16).

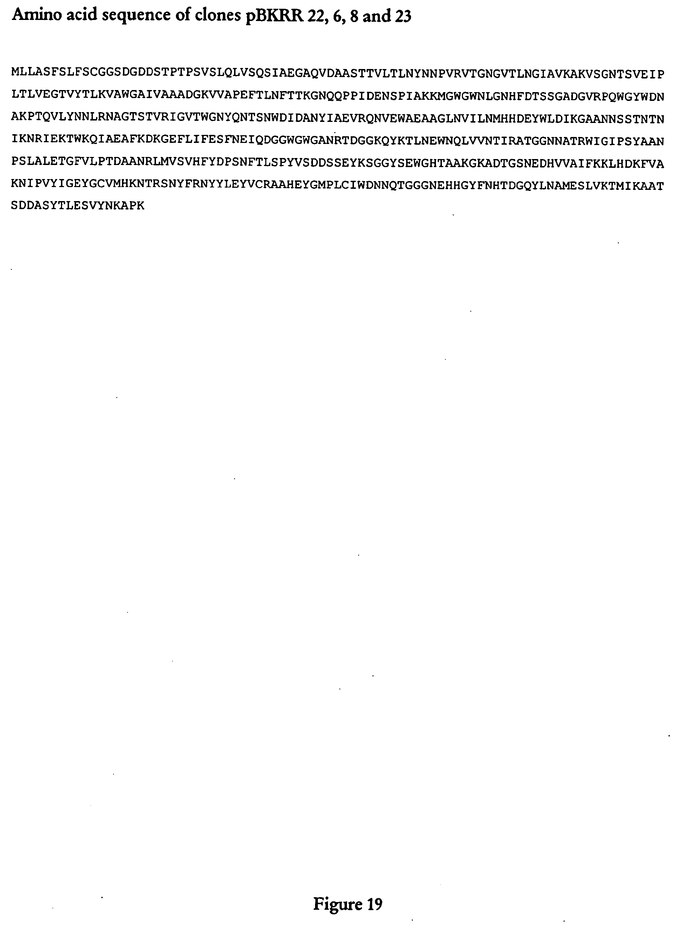

**Figure 19** represents the amino acid sequence of pBKRR 22, pBKRR 6, pBKRR 8 and pBKRR 23.

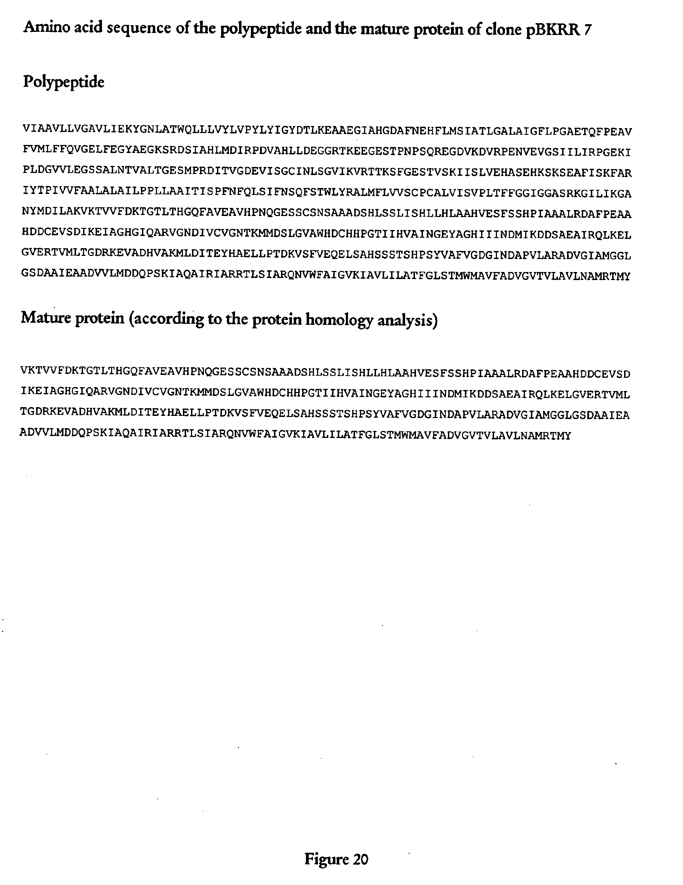

**Figure 20** represents the amino acid sequence of pBKRR 7 (polypeptide and mature protein).

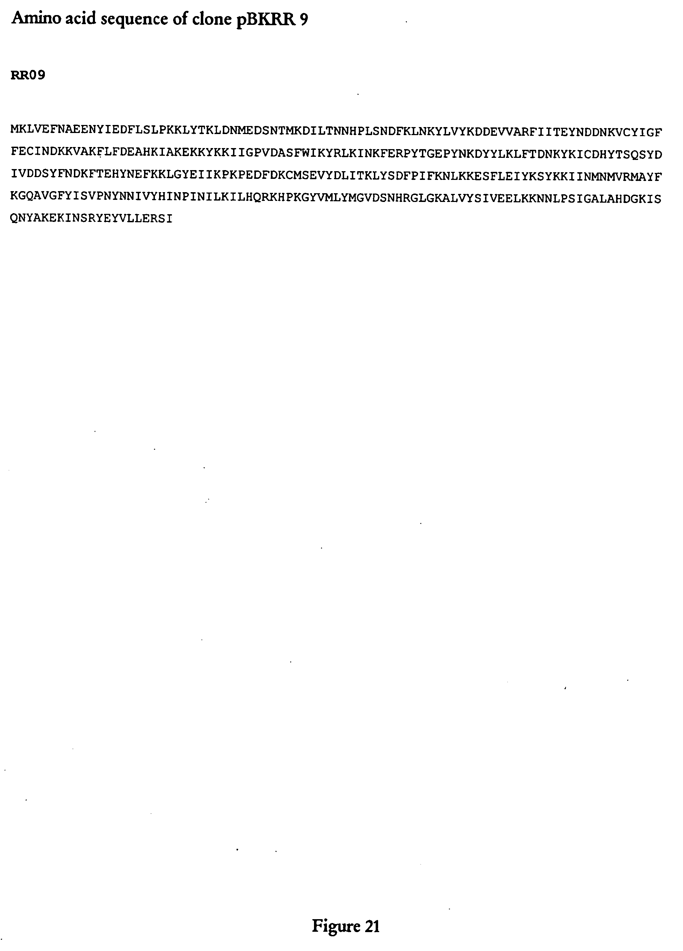

**Figure 21** represents the amino acid sequence of pBKRR 9.

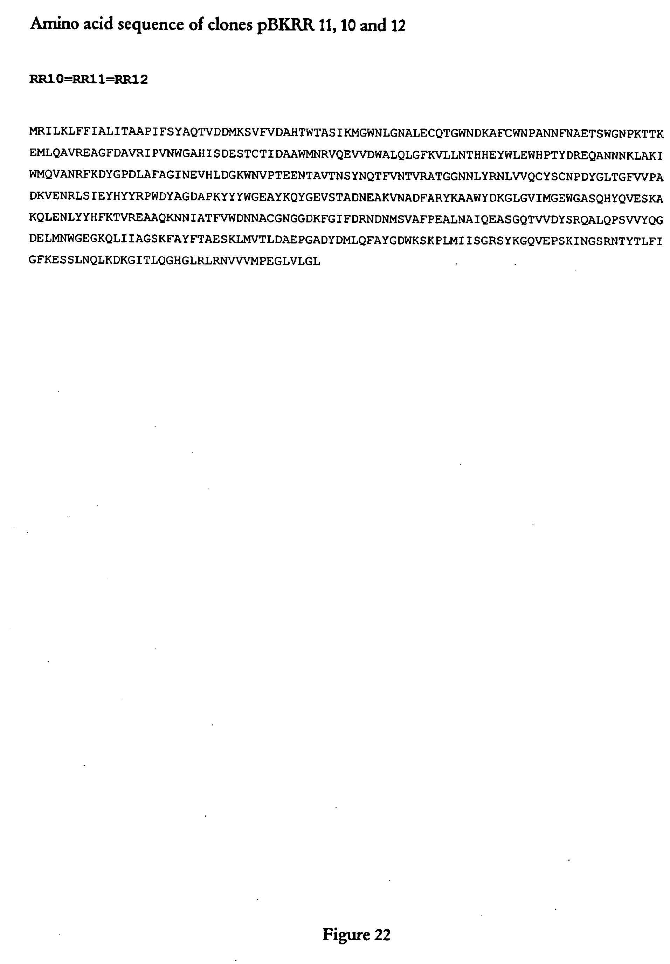

**Figure 22** represents the amino acid sequence of pBKRR 11, pBKRR 10 and pBKRR 12.

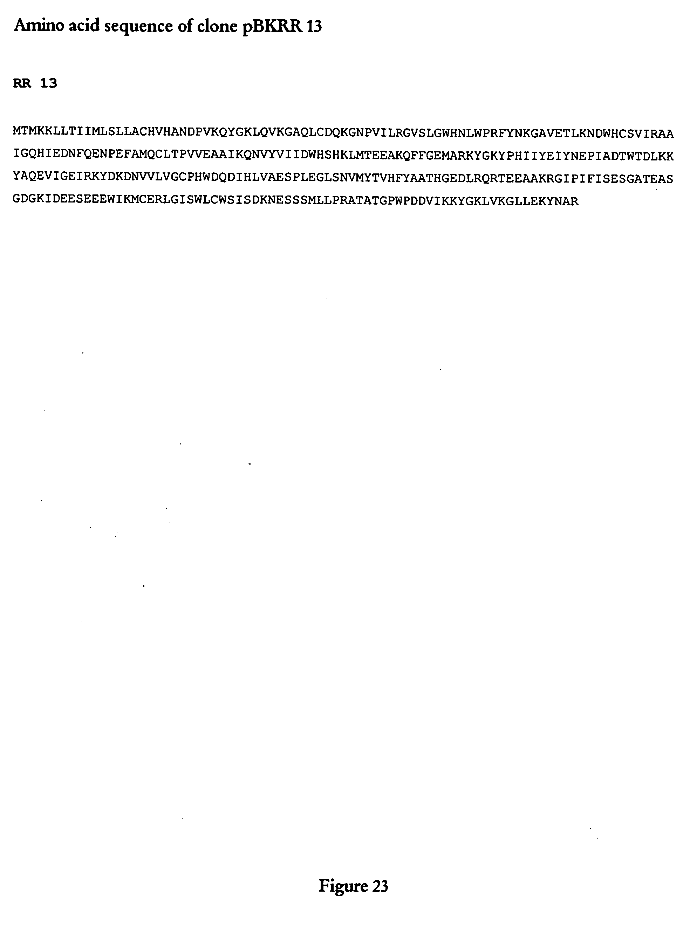

**Figure 23** represents the amino acid sequence of pBKRR 13 and

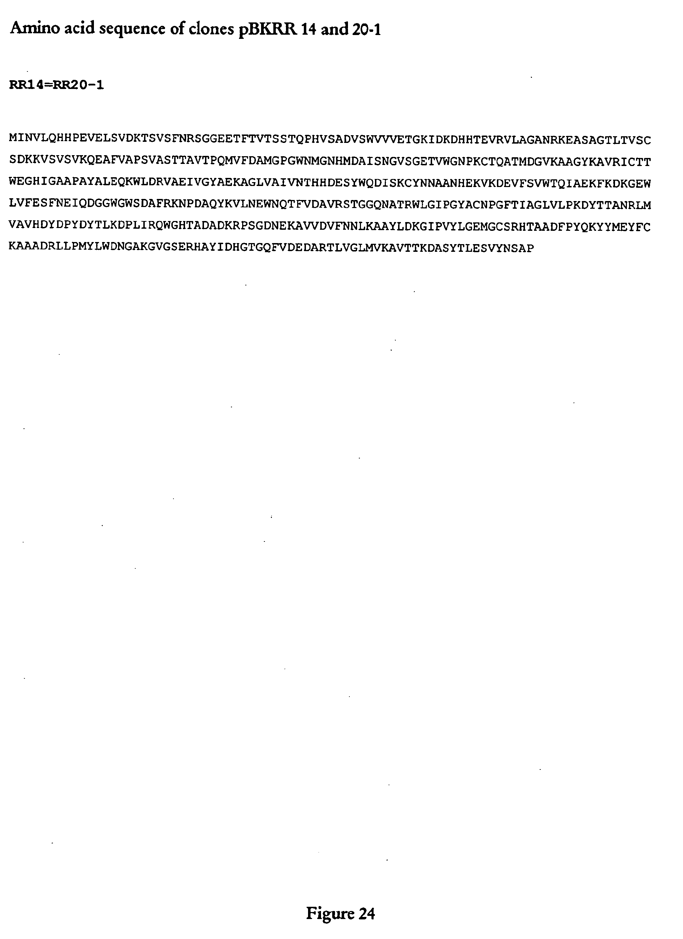

**Figure 24** represents the amino acid sequence of pBKRR 14 and pBKRR 20-1.

### Examples

### Materials and buffers

Ostazin brilliant red-hydroxyethyl cellulose, potassium sodium tartrate, 3,5-dinitro-salicylic acid, carboxymethyl cellulose and Sigmacell® (type 101) were purchased from Sigma Chemical Co. (St. Louis, MO, USA). Hydroxyethyl-cellulose (medium viscosity), cellobiose, cellotriose, cellotetraose, cellopentaose and cellohexaose were purchased from Fluka (Oakville, ON).

Molecular mass markers for SDS- and native-PAGE were provided from Novagen and Amersham Pharmacia Biotech (Little Chalfont, United Kingdom), respectively. Restriction and modifying enzymes were obtained from New England Biolabs. DNase I grade II, was obtained from Boehringer Mannheim.

All other chemicals were of the highest grade commercially available. Unlike otherwise indicated, the standard buffer described here was 50 mM sodium acetate, pH 5.6.

### Example 1

### Production of a genomic DNA library from rumen ecosystem

A genomic DNA library from DNA purified directly from rumen ecosystem was generated as described previously (Ferrer et al., Molecular Biology (2004) 53 (1), pp. 167 - 182). Parallel to this the genomic DNA library was generated using the Stratagene Lambda ZAP Express Kit (Stratagene protocols, Catalog #239212, Catalog #239615 and Revision #053007) according to the manufacturers standard protocol.

### Example 2

### Endoglucanase discovery and expression screening

Approximately 50,000 clones from selected genomic DNA libraries were plated to produce plaques on semi-solid medium according to standard procedures (Sambrook J, Maniatis T (1989) Molucular Cloning, A laboratory manual, Cold Spring Harbor Laboratory Press, Cold Spring Harbor, New York). The library was chosen for endoglucanase discovery as follows: 25 ml of a solution 1% (w/v) Ostazin brilliant red-hydroxyethyl cellulose in NZY medium (sterilized by filtration in 0.22 µm filter disk) was added to 25 ml NZY soft-agar containing infected *E. coli* cells, after which the mixture was plated. The plates (22.5 x 22.5 cm) contained about 10000 phage clones, were incubated overnight (approx. 16 h). Positive plaques, identified by the appearance of a clearing zone or "halo", were then purified. From the selected phages, the pBK-CMV plasmids have been excised using co-infection with helper phage, f1 and transferred to *E. coli* XLOLR (according to Stratagene protocols, Catalog #239212, Catalog #239615 and Revision #053007). The cellulose encoding inserts were sequenced from both ends using universal primers T3 and T7. The inserts were sequenced furthermore by "primer walking approach" to sequence both strands of the insert completely.

### Example 3

### Subcloning and expression of endoglucanase genes

Endoglucanase genes from pBK-CMV plasmids obtained previously (Example 2) were expressed in *E. coli* XLOLR that were grown to mid-log phase and induced with 0.1 mM isopropyl-β -D-galactopyranoside for 2 h at 37°C. Cell growth rate was measured by absorbance at 600 nm using a Beckman DU-7400 spectrophotometer. Cells were resuspended in standard buffer and subjected to lyophilization. The subcloning and expression of the endoglucanase genes were performed according to the Stratagene protocols, Catalog #239212, Catalog #239615 and Revision #053007 (according to the manufacturers standard protocol).

### Example 4

### Recovery of endoglucanases

The endoglucanases were recovered from 1-liter shaked flask fermentations, in Luria-Bertany broth plus 50 µg of Kanamycin per ml. One gram of fermentation broth was mixed with 10 ml of standard buffer in a 50-ml Falcon tube. The solution was vortexed and then incubated with DNase I grade II, for 30-45 min, and then sonicated for 4 min total time. A sample of the soluble fraction was separated from insoluble debris by centrifugation (10,000 x g, 30 min, 4°C) and proteins were precipitated by addition of 80% chilled acetone (-20°C). Proteins were resuspended in standard buffer and stored at -20°C at a concentration of 5 mg/ml until use. The amount of protein expression was examined using SDS-PAGE with 10-12% acrylamide. Gels were stained with Coomasie Blue and the appropriate molecular weight region was examined for determination of endoglucanase protein compared with the total protein. Cellulases were purified by preparative non-denaturing PAGE (5-15% polyacrilamide) at 45 V constant power at 4°C according to the manufacturer's (Bio-Rad) protocol. The gel region containing the active cellulase, detected in a parallel track by activity staining (using Ostazin brillian-blue carboxymethyl cellulose) was excised, suspended in two volumes of standard fuffer and homogenized in a glass tissue homogenizer. The eluate obtained after removal of polyacrilamide by centrifugation at 4,500 g at 4°C for 15 min was concentrated by ultrafiltration on a Centricon YM-10 (Amicon, Millipore) to a total volume of 1000 µl. The sample was further purified on a Superose 12 HR 10/30 gel filtration column pre-equilibrated with standard buffer containing 150 mM NaCl. Separation was performed at 4°C at a flow rate of 0.5 ml/min.

### Example 5

### Cellulase activity assay

The standard cellulase activity was determined in a continuous spectrophotometric assay by measuring the release of reducing sugars from the 4% (wt/vol) carboxymethyl cellulose (CMC) in 0.5-ml reaction mixtures containing 50 mM sodium acetate buffer, pH 5.6, at 40°C, using the dinitrosalycilic acid (DNS) method (Bernfeld, P. (1955) Amilases and b. *In:* Colowick SP, Kaplan NO (eds). Methods in Enzymology, Academic Press, New York, pp. 133-155). The reaction was performed in 0.5 ml scale containing 20 mg substrate and 5 mg lyophilised cells or 0.1 mg purified enzyme. Reactions were allow to proceed for a time period of 5-30 min after which the samples were centrifuge (10,000 x g, 3 min). For determination of reducing sugars, 50 µl of each sample was taken and mixed with 50 µl of dinitrosalycilic acid (DNS) solution (0.2 g DNS + 4 ml NaOH (0.8g/10ml) + 10 ml H2O + 6 g potassium sodium tartrate) in 96 -well microtiter plates. The samples were heated at 95°C for 30 min and cooled to room temperature. Finally, each well was diluted with 150 µl water and the absorbance at 550 nm was measured. Hydrolytic activity towards others cellulose substrates, i.e. hydroxyethylcellulose, Sigmacell and acid -swollen cellulose, laminarin and barley glucan was performed using conditions described for the standard cellulose assay, as described above. Reaction mixtures were mixed by mechanical agitation at 1,000 rpm. All enzyme assays were determined to be linear with respect to time and protein concentration. Sample blanks were used to correct for non-enzymatic release of the reduced sugar. One unit is defined as the amount of enzyme liberating 1 µmol of glucose-equivalent reducing groups per minute. Non-enzymatic hydrolysis of the substrates at elevated temperatures was corrected for with the appropiate blanks.

The products formed by hydrolysis of cellulose-based substrates (cellobiose, cellotetraose, cellopentaose, cellulose and carboxymethyl cellulose) were analysed by thin-layer chromatography (TLC). Solutions containing 20 µg of *E*. *coli* cell lysate proteins and 0.2% (wt/vol) substrate in 50 mM acetate buffer (pH 5.6) were incubated at 50°C until maximum conversion was achieved. The reactions were terminated by heating the preparation for 5 min at 95°C and hydrolysis products were separated by TLC on silica gel plates (Merck, Darmstadt, Germany) by using a mixture chloroform, glacial acetic acid, and water (6:14:1) as the solvent. Glucose, cellobiose and cellotriose were used as standard for the identification of the hydrolysis products. Sugars were visualized by heating 105°C for 15 min the plates which were previously sprayed with a reagent containing aniline (2 ml), diphenylamine (2 g), acetone (100 ml) and 85% H3PO4 (15 ml).

Hydrolytic activity using *p*-nitrophenyl derivatives as substrates was assayed spectrophotometrically at 405 nm, in 96-well microtiter plates. Briefly, the enzyme activity was assayed by the addition of 5 µl enzyme solution (50 µM) to 5 µl of 32 mM *p*-nitrophenyl glycosides (Sigma) stock solution (in acetonitrile), in 2.850 µl 50 mM sodium acetate buffer, pH 5.6. Reaction was allow to proceed from 2 to 300 min, at 40°C and the hydrolytic reaction was monitored. One unit of enzymatic activity was defined as the amount of protein releasing 1 µmol of *p*-nitrophenoxide/min from *p*-nitrophenyl glycoside at the indicated temperature and pH.

The optimal pH for enzyme activity was measured by incubating the enzyme substrate mixture at pH values ranging from 5.5 to 10.5 for 5-30 min at 40°C, using the standard method described above and CMC as substrate. The different buffers used were sodium citrate (pH 3.5-4.5), sodium acetate (pH 5.0-6.0), HEPES (pH 7.0), Tris -HCl (pH 8.0-9.0) and glycine-NaOH (pH 9.0-10.5), at a concentration of 50 mM. For temperature-dependent assay 50 mM sodium acetate buffer pH 5.6, was chosen. The optimal temperature for activity was determined quantitatively after incubating the enzyme substrate (CMC) mixture at temperatures ranging from 4 to 80°C. The solution was allowed to proceed for 3-30 min after which the enzymatic activity was measured as described above. In a second set of experiments, the pH and thermal stability was determined in the presence (40 g/l) or absence of calcium by prein-cubating the enzyme at pH from 3.5 to 10.5 and temperatures ranging from 30 to 80°C. 5 µl aliquots were withdrawn at times and remaining cellulase activity was measured at 40°C using the standard assay as described above. Activity pre- and postincubation was measured to calculate residual activity.

### Example 6

### Effect of various chemicals on cellulase activity

To study the effect of cations, inhibitors and surfactants on endoglucanase activity, the conditions were as follows: All cations tested were added as chloride salts and tested at a concentration of 10 mM. Inhibitors such as N-ethylmaleimide, iodoacetate and *p*-chloromercuribenzoate at concentration from 1-5 mM were incubated in the presence of the enzyme for 30 min (30°C) prior to the addition of the substrate (CMC). The effects of detergents on the endoglucanase activity was analysed by adding of 1-3% (wt/vol) detergent to the enzyme solution. In all cases, the enzyme activity was assayed in triplicate using the standard cellulose assay described above and the residual activity was expressed as percent of the control value (without addition of chemicals).

### Example 7

### Protein determination and N-terminal and internal amino acid sequence

Concentrations of soluble proteins were determined by the Bradford dye-binding method with a Bio-Rad Protein Assay Kit with bovine serum albumin as standard (Bradford, MM (1976) A rapid and sensitive method for the quantification of microgram quantities of protein utilizing the principle of protein-dye binding, Anal Biochem 72:248-254.). SDS-PAGE and native electrophoresis were performed according to Laemmli, UK (1970), Cleavage of structural proteins during the assembly of the head of bacteriophage T4, Nature 227:680-685. For NH₂-terminal amino acid sequencing, the purified proteins were subjected to PAGE in the presence of sodium dodecyl sulfate (SDS) and protein bands were blotted to a polyvinylidene difluoride membrane (Millipore Corp.) using semidry blot transfer apparatus according to the manufacturer's instructions. The blotted membrane was stained with Coomassie Brilliant Blue R250, and after destaining with 40% methanol/10% acetic acid the bands were cut out and processed for N-terminal amino acid sequence. The internal sequence was determined as follows: after SDS-PAGE (10-15% acrylamide) the protein bands stained with Coomassie brilliant blue R-250 were cut out and digested with trypsin and then sequenced using a protein sequencer (Bruker Daltonics).

**Table of Correspondence**

| | |
|---|---|
| **SEQ ID NO** | **Support** |
| 1 | Fig No. 9 |
| 2 | Fig No. 9 |
| 3 | Fig No. 10 |
| 4 | Fig No. 10 |
| 5 | Fig No. 11 |
| 6 | Fig No. 12 |
| 7 | Fig No. 13 |
| 8 | Fig No. 14 |
| 9 | Fig No. 15 |
| 10 | Fig No. 16 |
| 11 | Fig No. 17 |
| 12 | Fig No. 17 |
| 13 | Fig No. 18 |
| 14 | Fig No. 18 |
| 15 | Fig No. 19 |
| 16 | Fig No. 20 |
| 17 | Fig No. 20 |
| 18 | Fig No. 21 |
| 19 | Fig No. 22 |
| 20 | Fig No. 23 |
| 21 | Fig No. 24 |

## Claims

1. Polypeptide comprising one of the amino acid sequences of amino acids No. 175 to No. 210 of the amino acid sequences shown in Figures 17 to 24 or a functional fragment, or functional derivative thereof.

2. Polypeptide of claim 1, wherein the polypeptide comprises one of the amino acid sequences of amino acids No. 175 to 210, preferably No. 170 to No. 220, more preferably No. 150 to No. 240, most preferably No. 120 to 280 of the amino acid sequences, shown in Figures 17 to 24.

3. Polypeptide of claim 1, wherein the polypeptide comprises one of the amino acid sequences shown in Figures 17 to 24 or a functional fragment, or functional derivative thereof.

4. Polypeptide of any of claims 1 to 3, wherein the polypeptide is derived from rumen, particularly from rumen ecosystem, preferably from cow rumen, more preferably from New Zealand dairy cow.

5. Polypeptide of any of claims 1 to 4, wherein the polypeptide shows activity at pH optimum, preferably at pH ranging from 3.5 to 10.0, more preferably from 3.5 to 5.5 or from 5.5 to 7.0 or from 6.5 to 9.0, most preferably from 7.0 to 10.0,

6. Polypeptide of any of claims 1 to 5, wherein the polypeptide shows activity at temperature optimum, preferably at a temperature from 40°C to 70°C, more preferably 40°C to 60°C, most preferably from 50°C to 70°C.

7. Polypeptide of any of claims 1 to 3, wherein the polypeptide shows activity at low addition of cations , preferably without any addition of cations.

8. Polypeptide of any of claims 1 to 6, wherein the polypeptide shows high specific activity towards its substrate.

9. Polypeptide of any of claims 1 to 7, wherein the polypeptide shows high stability towards its substrate.

10. Polypeptide of any of claims 1 to 8, wherein the polypeptide shows a combination of at least two features, preferably three features, more preferably four features, most preferably five features of claims 4 to 8.

11. A nucleic acid encoding a polypeptide of any of claims 1 to 9 or a functional fragment or functional derivative thereof.

12. The nucleic acid of claim 11 comprising or consisting of one of the nucleic acid sequences of Figure 9 to 16.

13. A vector comprising the nucleic acid of claim 10 or 11.

14. A host cell comprising the vector of claim 12 and/or the nucleic acid of claim 10 or 11.

15. A method for the production of the polypeptide of any of claims 1 to 9 comprising the following steps:
a. cultivating a host cell of claim 13 and expressing the nucleic acid under suitable conditions;
b. isolating the polypeptide with suitable means.

16. Use of the polypeptide of any of claims 1 to 9 and/or the nucleic acid of claim 10 or 11 for the treatment of cellulosic textiles or fabrics, e.g. as an indegrient in detergent compositions or fabric softener compositions.

17. Use of the polypeptide of any of claims 1 to 9 and/or the nucleic acid of claim 10 or 11 in the treatment of paper pulp.

18. Use of the polypeptide of any of claims 1 to 9 and/or the nucleic acid of claim 10 or 11 in consumer products, particularly food products.

19. Use of the polypeptide of any of claims 1 to 9 and/or the nucleic acid of claim 10 or 11 as an additive for animal food.

20. Use of the polypeptide of any of claims 1 to 9 and/or the nucleic acid of claim 10 or 11 as a protoplast preparation agent, a herbicide or a drench in ruminants.

21. Use of the polypeptide of any of claims 1 to 9 and/or the nucleic acid of claim 10 or 11 in the production of anti-cellulase reagents.
